# EUROPEAN PATENT SPECIFICATION

(11) **EP 1 290 224 B1**
(45) Date of publication and mention of the grant of the patent: **25.08.2010**
(21) Application number: 01936994.1
(22) Date of filing: 30.05.2001
(51) Int. Cl.: C12Q 1/68

(54) **DIAGNOSIS KIT FOR MYCOBACTERIUM SPECIES IDENTIFICATION AND DRUG-RESISTANCE DETECTION AND MANUFACTURING METHOD THEREOF**
DIAGNOSTISCHES KIT ZUR IDENTIFIKATION UND FESTSTELLUNG VON WIRKSTOFFRESISTENZ BEI MYKOBAKTERIUM SOWIE HERSTELLUNGSMETHODEN DAVON
KIT DE DIAGNOSTIC POUR L'IDENTIFICATION DE L'ESPECE MYCOBACTERIUM ET LA DETECTION DE LA RESISTANCE AUX MEDICAMENTS, ET SON PROCEDE DE FABRICATION

(30) Priority: 30.05.2000 KR 2000029369
(43) Date of publication of application: 12.03.2003
(73) Proprietor: Biomedlab Co., Ltd., Seoul 110-510 (KR)
(72) Inventor: Kim, Hyun-Jung, Bundang-gu Songnam-city 463-010 (KR); Kim, Na-Young, Yangchun-gu, Seoul 158-055 (KR); Yoon, Sung-Wook, Gangnam-gu, Seoul 135-270 (KR); Kim, Jeong-Mi, Eunpyong-gu, Seoul 122-041 (KR); Park, Mi-Sun, Busan 617-010 (KR)
(74) Representative: Keller, Günter
(86) International application number: PCT/KR2001/000904
(87) International publication number: WO 2001/092573

(56) References cited:
- EP-A2- 1 076 099
- WO-A-95/33851
- WO-A-97/29212
- LEE H ET AL: "Molecular analysis of rifampin-resistant Mycobacterium tuberculosis isolated from Korea by polymerase chain reaction-single strand conformation polymorphism sequence analysis." THE INTERNATIONAL JOURNAL OF TUBERCULOSIS AND LUNG DISEASE : THE OFFICIAL JOURNAL OF THE INTERNATIONAL UNION AGAINST TUBERCULOSIS AND LUNG DISEASE. JUL 1998, vol. 2, no. 7, July 1998 (1998-07), pages 585-589, XP009063966 ISSN: 1027-3719
- ZAMMATTEO NATHALIE ET AL: "Comparison between different strategies of covalent attachment of DNA to glass surfaces to build DNA microarrays" ANALYTICAL BIOCHEMISTRY, ACADEMIC PRESS, SAN DIEGO, CA, US, vol. 280, no. 1, 10 April 2000 (2000-04-10), pages 143-150, XP002159089 ISSN: 0003-2697
- MILLER L P ET AL: "THE RPOB GENE OF MYCOBACTERIUM TUBERCULOSIS" ANTIMICROBIAL AGENTS AND CHEMOTHERAPY, AMERICAN SOCIETY FOR MICROBIOLOGY, WASHINGTON, DC, US, vol. 38, no. 4, April 1994 (1994-04), pages 805-811, XP009015588 ISSN: 0066-4804
- WILLIAMS D L ET AL: "Contribution of rpoB mutations to development of rifamycin cross-resistance in Mycobacterium tuberculosis." ANTIMICROBIAL AGENTS AND CHEMOTHERAPY. JUL 1998, vol. 42, no. 7, July 1998 (1998-07), pages 1853-1857, XP002374758 ISSN: 0066-4804
- KIM B J ET AL: "IDENTIFICATION OF MYCOBACTERIAL SPECIES BY COMPARATIVE SEQUENCE ANALYSIS OF THE RNA POLYMERASE GENE (RPO B)" JOURNAL OF CLINICAL MICROBIOLOGY, WASHINGTON, DC, US, vol. 37, no. 6, June 1999 (1999-06), pages 1714-1720, XP000913990 ISSN: 0095-1137
- THOMAS T. GINGERAS ET AL.: 'Simultaneous genotyping and species identification using hybridization pattern recognition analysis of generic mycobacterium DNA arrays' GENOME RESEARCH vol. 8, no. 5, 1998, pages 435 - 448, XP002938600
- TROESCH A. ET AL.: 'Mycobacterium species identification and rifampin resistance testing with high-density DNA probe array' JOURNAL OF CLINICAL MICROBIOLOGY vol. 37, no. 1, 1999, pages 49 - 55, XP002130858
- VICTOR T.C. ET AL.: 'Detection of mutation in drug resistance genes of mycobacterium tuberculosis by a dot-blot hybridization strategy' TUBERCLE AND LUNG DISEASE vol. 79, no. 6, 1999, pages 343 - 348, XP002960363
- ROSSAU R. ET AL.: 'Evaluation of the INNO-LiPA RiF. TB assay, a reverse hybridization assay for the simultaneous detection of mycobacterium tuberculosis complex and its resistance to rifampin' ANTIMICROB. AGENTS CHEMOTHER. vol. 41, no. 10, 1997, pages 2093 - 2098, XP002960364

## Description

### Technical Field

The present disclosure relates to a diagnosis kit for Mycobacterium species identification and drug-resistance detection and a manufacturing method thereof, and more specifically, to a diagnosis kit for Mycobacterium species identification and drug-resistance detection in which point mutations of Mycobacterial rpoB gene related to the drug-resistance can be discriminated speedily and accurately in large quantity by using an oligonucleotide chip, and a manufacturing method thereof.

### Background Art

About two million people die from tuberculosis worldwide each year. The increase in immigration, the spread of HIV/AIDS, and the emergence of drug-resistance strains are enhancing the mortality of tuberculosis. AIDS patients and newborns with weak immune system can develop tuberculosis from not only Mycobacterium tuberculosis infection but also MOTT (Mycobacterium other than tuberculosis) infection, particularly, Mycobacterium avium-intracellulare (MAI), Mycobacterium chelonae, Mycobacterium fortuitum, Mycobacterium kansasaii, Mycobacterium xenopi, Mycobacterium marinum, Mycobacterium scrofulaceum, and Mycobacterium szulgai.

Tuberculosis is normally treated by chemotherapy with various anti-tuberculosis drugs. Since there are numerous different strains of Mycobacteria with diverse drug-susceptibility, detection and identification of the causative bacterium is important for the effective treatment.

For the diagnosis of tuberculosis, chest X-ray examination and sputum test are commonly used. However, chest X-ray can often misdiagnose an already-cured inactive tuberculosis or other types of chest diseases as tuberculosis. Sputum test can be typically preformed by the following two methods: sputum smear microscopy in which sputum of a patient is thinly spread on a slide, and then the bacteria are selectively stained for microscopic observation, and culture method in which the bacteria are cultured under physiological conditions for visual observation. Sputum smear microscopy is relatively simple and rapid, but cannot easily discriminate Mycobacterium tuberculosis from MOTT. Detection and identification of the mycobacterium strains have been done by the culture method, but it takes at least 6-8 weeks due to the slow growth rate of the bacteria. During this period, a combination of primary anti-tuberculosis drugs is used, which may result in therapeutic failure and drug-resistance. In fact, increasing numbers of strains have been determined to be resistant to primary anti-tuberculosis drugs such as isoniazid (INH), rifampin (RIF), streptomycin (STR), ethambutol (EMB), pyrasinamide (PZA).

Therefore, in order to achieve effective control and treatment of tuberculosis, a necessity of rapidly identifying the causative bacterium at the initial stage of infection has been increasingly recognized. Recently, molecular biological techniques by which mycobacterial species identification can be made within a relatively short time have been devised. These methods use species-specific sequences of the mycobacterial genes such as IS6110 as the target DNA. However, the IS6110 gene has different copy numbers in various mycobacterial species and does not present in some species. Several reports indicate that the IS6110 gene is not mycobacterium-specific. Therefore, the use of IS6110 as target DNA may cause false-positive or false-negative results. The highly polymorphic region of 16S rRNA DNA has also been used for species identification since it shows species-specific polymorphism. Several commercial products have been developed based on this method: Accuprobe (Gen-probe, San Diego, CA, U.S.A.) which can be applied directly to a specimen, and AMTD (Gen-probe, San Diego, CA, U.S.A.) and Amplicor MTB (Roche Diagnostics Systems, Somerville, NJ, U.S.A.) in which target DNA are PCR (polymerase chain reaction) amplified prior to analysis to enhance sensitivity. However, the 16S rRNA gene occasionally has different copies in a single strain, and this could complicate accurate species determination. Moreover, in order to assess the drug-resistance, an additional molecular biological analysis on the rpoB gene has been necessary.

Hereinafter, point mutations of Mycobacterium tuberculosis related to drug-resistance are described in detail. Resistance against RIF, a primary anti-tuberculosis drug, is known to be related to the mutations of rpoB gene encoding RNA polymerase β -subunit. More than 30 different mutations identified so far have shown to be concentrated in the 81 bp core region of the 3,534bp rpoB gene (Amalio Telenti, Paul Imboden, Francine Marchesi, Douglas Lowrie, Sterwart Cole, M. Joseph Colston, Lukas Matter, Kurt Schopfer, and Thomas Bodmer, "Detection of rifampin-resistance mutation in Mycobacterium tuberculosis" THE LANCET, Vol.341, pp. 647-650, 1993).

Table 1 illustrates point mutations occurring in codons 507 through 533 of the M. tuberculosis rpoB gene. As illustrated in Table 1, point mutations occur more frequently in codons 513, 516, 526, and 531, which are also observed more prevalently than the others.

**[Table 1]**

| CODON | VARIANT NUCLEOTIDE (AMINO ACID CHANGE) |
|---|---|
| 510 | CAG(Gln) > CAT(His) |
| 511 | CTG(Leu) > CCG(Pro), CGG(Arg) |
| 512 | AGC(Ser) > ACC(Thr), CGC(Arg) |
| 513 | CAA(Gln) > CTA(Leu), AAA(Lys), CCA(Pro) |
| 515 | ATG(Met) > ATA(Ile) |
| 516 | GAC(Asp) > GTC(Val), TAC(Tyr), GAG(Glu), GGC(Gly) |
| 521 | CTG(Leu) > ATG(Met) |
| 522 | TCG(Ser) > TTG(Leu) |
| 526 | CAC(His) > TAC(Tyr), GAC(Asp), CGC(Arg), CTC(Leu), CCC(Pro), CAA(Glu), AAC(Asn), CAG(Gln) |
| 531 | TCG(Ser) > TTG(Leu), TGG(Trp), TGT(Cys), CAG(Gln) |

Rifabutin (abbreviated as RIB), a spiro-piperidyl derivative of rifamycin S, is used to prevent MAC infection in AIDS patients (James M. Musser, "Antimicrobial Agent Resistance in Mycobactera: Molecular Genetic Insights", Clinical Microbiology Review, Vol. 8, No. 4, pp. 496-514, 1995). Resistance against RIB is also related to the point mutations of the rpoB gene. However, the drug-susceptibility of a mutation displays difference between RIF and RIB: Several mutations showing resistance to RIF have determined to be susceptible to RIB. Mutation showing resistance to both RIF and RIB were also reported (Della Bruna C., G. Schiopacassi, D. Ungheri, D. Jabes, E. Morvillo, and A. Sanfilippo, "A new spiro-piperidyl rifamycin: in vitro and in vivo studies", J. Antibiot. Vol. 36, pp. 1502-1506, 1983, Dessen A., A. Quenmard, J. S. Blanchard, w. R. Jacobs, Jr., and J. C. Sacchettini, "Crystal structure and function of the isoniazid target of Mycobacterium tuberculosis" Science, Vol. 267, pp. 1638-1641, 1995). RIB has often been used to treat RIF-resistant infection, which were effective in many cases (Gillespie S. H., A. J. Baskerville, R. N. Davidson, D. Felmingham, and A. D. M. Bryceson, "The serum rifabutin concentrations in patient successfully treated for multi-resistant Mycobacterium tuberculosis infection", J. Antimicrobi. Chemother. Vol. 33, pp. 661-674, 1990).

In addition, the species-specific characteristics of rpoB gene have been used for mycobacterial species identification. Korean Patent Registration No. 234975 discloses a method for the detection and identification of mycobacterial strains by PCR-RFLP (restriction fragment length polymorphism after polymerase chain reaction) assay using a set of novel PCR primers that specifically amplifies the rpoB gene of mycobacterial species. Also, Korean Patent Registration No. 285254 discloses a method of detecting and distinguishing Mycobacterium tuberculosis complex and MOTT by using primers that can amplify rpoB gene fragments of Mycobacterium tuberculosis complex and MOTT in a respectively different size.

However, the above-described methods require bacterial culture step prior to PCR amplification, limit the kind of species or the number of samples that can be analyzed, and are laborious having a PCR step followed by a restriction enzyme treatment step and/or an electrophoresis step.

In the Korean Patent Registration No. 285253, a method of detecting rifampin-resistance in Mycobacterium tuberculosis with a nested PCR-SSCP (polymerase chain reaction-single strand conformational polymorphism) and a single-step nested PCR-SSCP. This method include two PCR and polymorphism assay steps and therefore it takes about four days to determine rifampin-resistance.

PCT International Publication No. WO97/29212 by Affymetrix discloses a technology of a sequencing DNA chip of rpoB gene. With this method, the entire base sequences of 700bp in the rpoB gene should be analyzes to obtain clinically important information such as species identity and drug-resistance.

B.-J. Kim et al. investigated the rpoB gene including the β-subunit of RNA polymerase. rpoB DNA sequences of 342bp were amplified from 44 reference strains of Mycobacteria and clinical isolates (107 strains) by PCR (Kim B.-J. et al., "Identification of Mycobacterial Species by Comparative Sequence Analysis of the RNA Polymerase Gene (rpoB)" Journal of Clinical Microbiology, vol. 37, No. 6, June 1999, pages 1714-1720).

In WO 95/33051 a method for the detection of the antibiotic resistance spectrum of Mycobacterium species is disclosed. The document discloses various probes for detecting resistance-conferring rpoB genes. However, there is still a need for probes having increased efficiency of detection and reducing the amount of time required for PCR.

H. Lee et al. describes amplification of a 157bp PCR product (page 586, left-hand column, PCR amplification). However, the focus of this document is on single-stranded conformation polymorphism sequence analysis and PCR sequence analysis. There is no teaching that hybridization analysis should be performed, or that an oligonucleotide chip containing specific sequences should be prepared (Lee H. et al., "Molecular Analysis of rifampin-resistant Mycobacterium Tuberculosis Isolated from Korea by Polymerase Chain Reaction - Single-Strand Conformation Polymorphism Sequence Analysis", The International Journal of Tuberculosis and Lang Disease, July 1998, vol. 2, No. 7, pages 585-589).

T. R. Gingeras et al. investigated an array designed to determine the specific nucleotide sequence of the rpoB gene of Mycobacterium tuberculosis with base sequences of 705bp accurately detected rifampin-resistance associated with mutations of 44 clinical isolates of M. tuberculosis (Thomas R. Gingeras et al., "Simultaneous Genotyping and Species Identification Using Hybridization Pattern Recognition Analysis of Generic Mycobacterium DNA Arrays", Genome Research 1998, vol. 8, No. 5, pages 435-448).

A. Troesch et al. describe the use of a DNA probe array based on two sequence databases: One for the species identification of Mycobacteria and the other for detecting Mycobacterium tuberculosis rifampin-resistance (A. Troesch et al., "Mycobacterium Species Identification and Rifampin-resistance Testing With High-density DNA Probe Array", Journal of Clinical Microbiology 1999, vol. 37, No. 1, pages 49-55).

In EP 1 076 099 a kit for diagnosis of tubercle bacilli is described.

T. C. Victor et al. describes the detection of mutations in drug-resistance genes of Mycobacterium tuberculosis by a dot-blot hybridization strategy (Victor T. C. et al., Tubercle and Lang Disease 1999, vol. 79, No. 6, pages 343-348).

R. Rossau et al. investigated the simultaneous detection of Mycobacterium tuberculosis complex and its resistance to rifampin with the INNO-LiPA Rif. TB assay, a reverse hybridization assay (R. Rossau, Antimicrobial Agents and Chemotherapy, October 1997, vol. 41, No. 10, pages 2093-2098).

D. L. Williams et al. investigated the contribution of rpoB mutations to development of rifamycin cross-resistance in Mycobacterium tuberculosis (D. L. Williams et al., Antimicrobial Agents and Chemotherapy, July 1998, vol. 42, No. 7, pages 1853-1857).

### Disclosure of the Invention

To solve the above problems, it is an object of the present invention to provide a diagnosis kit for Mycobacterium species identification and drug-resistance detection in which features relating to both the Mycobacterium species identification and the drug-resistance detection can be discriminated speedily and accurately in large quantity.

Further described herein is a method of manufacturing a diagnosis kit for Mycobacterium species identification and drug-resistance detection in which features relating to both the Mycobacterium species identification and the drug-resistance detection can be discriminated speedily and accurately in large quantity.

Still further described herein is a set of primers of a polymerase chain reaction (PCR) for faster Mycobacterium species identification by enabling efficient PCR amplification of rpoB gene fragment (157bp) of numerous species directly from uncultured specimens including sputum samples.

Also described herein are experimental conditions for polymerase chain reaction (PCR) for efficient amplification of the 157bp fragment of rpoB gene from various Mycobacteria.

The present invention is defined by the claims.

Generally, it is described a diagnosis kit for Mycobacterium species identification and drug- resistance detection comprising: an oligonucleotide chip including species identification probes comprised of species-specific DNA sequences of Mycobacterial rpoB gene, Mycobacterial drug-resistance detection probes comprised of one or more modified codons of Mycobacterial rpoB gene, and control group probes comprised of wild-type sequences corresponding to each drug-resistance detection probe; and a method for detecting the hybridization of the oligonucleotide chip and the specimen DNA.

By the above construction, drug-resistance detection as well as Mycobacterium species identification can be simultaneously performed. In particular, since Mycobacterial rpoB gene point mutations related to drug-resistance can be discriminated by comparing the intensity of hybridization signal of the drug-resistance detection probe with that of the control group probe, and species identification can be made by comparing the hybridization intensities of the species-specific probes, the Mycobacterial species identification and the drug-resistance detection can be performed speedily and accurately in large quantity.

In said diagnosis kit for Mycobacterial species identification and drug-resistance detection, the drug-resistance detection probe may comprise one or more modified codons discovered in 507-533 codons ofrpoB gene.

By the above construction, since one or more modified codons discovered in 507-533 codons in which point mutations frequently occur are used as a probe, drug-resistance can be discriminated speedily and accurately with only part of the Mycobacterial rpoB gene.

In said diagnosis kit for Mycobacterial species identification and drug-resistance detection, the drug-resistance detection probes can be rifampin-resistance probes and/or rifabutin-sensitive probes.

By the above construction, it can be discriminated speedily and accurately whether a specimen has rifampin-resistance and/or rifabutin susceptibility.

According to another embodiment of the present disclosure, there is provided a method of manufacturing a diagnosis kit for Mycobacterium species identification and drug-resistance detection comprising the steps of: (a) modifying species identification probes comprised of species-specific DNA sequences of Mycobacterial rpoB gene, Mycobacterial drug-resistance detection probes comprised of one or more modified codons of Mycobacterial rpoB gene, and contrast group probes comprised of wild-type sequences corresponding to each drug-resistance detection probe to contain an amine group on the 5' terminal; (b) inducing an aldehyde group on glass; and (c) fabricating an oligonucleotide chip by immobilizing the modified probes on the glass by Schiff base reaction.

By the above construction, the diagnosis kit having the advantage of simultaneously discriminating Mycobacterium species identity and drug-resistance speedily and accurately in large quantity, can be easily fabricated.

Further described herein is a set of PCR primers for Mycobacterium species identification comprises DGR8 and DGR9 having base sequences by which Mycobacterial rpoB gene fragments (157bp) are specifically amplified.

In the case that the primer set is used, DNA isolated from uncultured clinical specimens can be used as a template of a polymerase chain reaction, and the rpoB gene fragment (157bp) of 44 species belonging to Mycobacterium genus can be efficiently amplified. Accordingly, the Mycobacterium species identification and the drug-resistance detection can be performed within a short time, for example, approximately in six hours. The PCR products obtained by using the primer set are DNA of 157bp size that can be used as a target DNA of oligonucleotide chip analysis.

In a method for Mycobacterium species identification and drug-resistance detection a fragment of rpoB gene in a specimen is PCR amplified under the reaction conditions such as an annealing temperature of 64°C, a reaction cycle of 40, and a primer concentration of 100pmol with primers DGR8 and DGR9, and then the amplified product is used for Mycobacterial species identification and drug-resistance detection by using said diagnosis kit according to the present invention.

DNA chip technology developed in early 1980s presents a method of providing a large amount of genetic information at a time. This method has been used to investigate gene expression, mutation and polymorphism of human or bacterium genome.

The present disclosure utilizes the DNA chip technology that enables rapid genetic analysis, and provides a diagnosis kit for Mycobacterium species identification and drug-resistance detection and a manufacturing method thereof, in which the Mycobacterium species identification and the point mutations of Mycobacterium tuberculosis related to drug-resistance can be discriminated.

Mutations occurring in the rpoB gene of Mycobacterium tuberculosis have been investigated to identify drug-resistant strains, based on the relationship between drug-susceptibility and rpoB gene mutation described in the background art. More specifically, the rpoB gene fragment (157bp) including the 81bp core region where Mycobacterial point mutations occur most frequently is PCR amplified using a particular primer set, and then the amplified DNA is hybridized with an oligonucleotide chip, to thereby detect point mutations for drug-resistance determination.

The method using said oligonucleotide chip includes a step of amplifying target DNA from clinical specimens by PCR. In order to successfully apply the method to clinical samples, PCR should be performed with highly efficient primers.

In Korean Patent Application No. 2000-0029369, biotin-TR8 and TR9 that have been devised by using only the rpoB gene base sequence of Mycobacterium tuberculosis are used as primers. The DNA amplified by using the primers is 157bp fragment containing the 81bop core region where point mutations related to rifampin-resistance occur most frequently. However, the amplification efficiency of biotin-TR8/ TR9 primers is not sufficient for uncultured specimens such as sputum and therefore the analysis time with these primers exceeds 6-8 weeks due to bacterial culture step. MOTT may not be amplified even from cultured samples due to the low efficiency of these primers. In other words, a rapid analysis on the species identity and drug-resistance cannot be accomplished without DNA amplification directly from sputum samples.

Thus, the inventors combine base sequences of the rpoB gene of forty-three different species of Mycobacteria as well as Mycobacterium tuberculosis, to thereby devise a new primer set. When this primer set is used, isolated DNA from uncultured specimens can be used as a template for PCR, and a specific rpoB gene sequence of forty-three bacterial species as well as Mycobacterium tuberculosis can be amplified by a single PCR step. When the amplified product is applied to a DNA chip, the Mycobacterium species identification and rpoB mutation detection can be simultaneously performed within a short period of time.

### Brief Description of the Drawings

FIG. 1 is a flowchart view illustrating a process of manufacturing an oligonucleotide chip for Mycobacterium species identification and drug-resistance detection according to the present invention;
FIG. 2 is a pictorial view showing a result in which rpoB gene is amplified using biotin-TR8 and TR9 primers and then undergone an electrophoresis in 2% agarose gel, according to the present invention;
FIGs. 3A through 3J are pictorial views showing fluorescent images obtained by hybridization of PCR amplified clinical samples using biotin-dUTP with the oligonucleotide chip, according to the present invention;
FIG. 4 is a view of an array format indicating the probe positions of the oligonucleotide chip used in the FIGs. 3A through 3J views;
FIG. 5 is a diagram showing an array format of an oligonucleotide chip using probes of SEQ ID NO 5 through 12 according to the present invention;
FIGs. 6A through 6C are pictorial views showing the experimental results of Mycobacterial species identification using the oligonucleotide chip of FIG. 5;
FIG. 7 is a diagram showing an array format of an oligonucleotide chip in which probes of base sequences of SEQ ID NO 41 through 46 detecting additional rpoB gene mutations related to rifampin-resistance in Mycobaterium tuberculosis are included;
FIGs. 8A through 8D are pictorial views showing the diagnostic results of clinical isolates regarding rifampin-resistance and rifabutin-susceptibility using the oligonucleotide chip of FIG. 7;
FIG. 9A is a pictorial view showing a 2% agarose gel DNA band image of PCR amplified products using PCR conditions disclosed in the prior application of the inventors, i.e. an annealing temperature of 63°C and 35 cycles with biotin-TR8 and TR9 primers of 50pmol;
FIG. 9B is a pictorial view showing a 2% agarose gel image of PCR amplified products using the PCR conditions according to the present invention, i.e. an annealing temperature of 64°C and 40 cycles with biotin-DGR8 and DGR9 primers of 100pmol;
FIG. 10 is a pictorial view showing a 2% agarose gel image of eleven sputum specimens PCR amplified by using biotin-DGR8 and DGR9 primers under the optimized condition according to the present invention; and
FIGs. 11A through 11B are pictorial views showing the diagnostic results of sputum specimens regarding rifampin-resistance using the oligonucleotide chip of FIG. 7.

### Best Mode for Carrying Out the Invention

Preferred embodiments will be described below in detail with reference to the accompanying drawings. However, the following embodiments are nothing but embodiments for demonstrating structures and effects of the present invention. Thus, the present invention is not limited to the following embodiments, but is limited by claims 1-14.

### Embodiment 1-1: Mycobacterial genomic DNA

Mycobacterial genomic DNA extracted from ten clinical isolate cultures were provided after sequencing from the Department of Respiratory Diseases, Asan Medical Center (Seoul, Korea), and additional Mycobacterial genomic DNA were obtained from ATCC (Manassas, VA USA).

### Embodiment 1-2: Fabrication of oligonucleotide probes and primers

Table 2 illustrates sequences of oligonucleotide probes and primers. Each of biotin-TR8 and TR9 and probes used were custom-synthesized from Bionics (Seoul, Korea). All other reagents were first-grade.

**[Table 2]**

| Probes or Primers | | Sequences |
|---|---|---|
| Tuberculosis bacteria group probes | | |
| | Mc1 | 5'Amine-T10-tcttcggcaccagccag 3' |
| | Mc2 | 5'Amine-T10-tcttcggaaccagccag 3' |
| | Mc5 | 5'Amine-T10-tcttcggaacgtcgcag 3' |
| | Mex | 5'Amine-Tl0-tcttcggaacctcgcag 3' |
| Mycobacterium species identification probes | | |
| | Ms1 | 5'Amine-T10-ggtctgtcacgtgagcgtg 3' |
| | Ms2 | 5'Amine-T10-ggtctgtcccgtgagcgtg 3' |
| | Ms3 | 5'Amine-T10-ggtctgtcgcgtgagcgtg 3' |
| | Ms4 | 5'Amine-T10-ggtctgtcccgggagcgtg 3' |
| | Ms5 | 5'Amine-T10-ggtctgtcccgcgagcgtg 3' |
| | Ms6 | 5'Amine-T10-ggtctgtcgcgcgagcgtg 3' |
| | Ms7 | 5'Amine-T10-ggtctgacccgtgaccgtg 3' |
| | Ms8 | 5'Amine-T10-ggtctgagccgggagcgtg 3' |
| Wild-type probes | | |
| | F2 | 5'Amine-T10-cagccagctgagccaat 3' |
| | F3 | 5'Amine-T10-gctgagccaattcatgg 3' |
| | F4 | 5'Amine-T10-attcatggaccagaaca 3' |
| | F5 | 5'Amine-T10-tggaccagaacaacccg 3' |
| | F6 | 5'Amine-T10-caacccgctgtcggggt 3' |
| | F7 | 5'Amine-T10-ggttgacccacaagcgc 3' |
| Rifampin-resistance probes | | 5'Amine-T10-ccgactgtcggcgctgg 3' |
| | mt511 | |
| | mt513 | 5'Amine-T10-cagccagccgagccaat 3' |
| | mt516a | 5'Amine-T10-gctgagcccattcatgg 3' |
| | mt516b | 5'Amine-T10-attcatggtccagaaca 3' |
| | mt518 | 5'Amine-T10-attcatgtaccagaaca 3' |
| | mt521 | 5'Amine-T10-tggaccagcacaacccg 3' |
| | mt522 | 5'Amine-T10-caacccgatgtcggggt 3' |
| | | 5'Amine-T10-caacccgctgttggggt 3' |
| | mt526b | 5'Amine-T10-ggttgacctacaagcgc 3' |
| | mt526c | 5'Amine-T10-ggttgaccgacaagcgc 3' |
| | mt531 | 5'Amine-T10-ggttgacccgcaagcgc 3' |
| | mt516c | 5'Amine-T10-ccgactgttggcgctgg 3' |
| | mt526d | 5'Amine-T10-attcatggagcagaaca 3' |
| | mt526e | 5'Amine-T10-ggttgaccaacaagcgc 3' |
| | mt526f | 5'Amine-T10-ggttgaccgccaagcgc 3' |
| | mt526g | 5'Amine-T10-ggttgacctgcaagcgc 3' |
| | mt526h | 5'Amine-T10-ggttgacccagaagcgc 3' |
| cf) Rifabutin-sensitive probes: | | 5'Amine-T10-ggttgacc'ggcaagcgc 3' |
| | Primers | |
| | Bio-TR 8 | 5'biotin-tgcacgtcgcggacctcc 3' |
| | TR 9 | 5'tcgccgcgatcaaggagt 3' |
| Mycobacterium species identification additional probes | | |
| | MTAB | 5'Amine-T10-gcagctgagccaattcat 3' |
| | MF | 5'Amine-a10-cgacgtcgcagctgtcg 3' |

According to the above order illustrated in Table 2, SEQ ID NOs 1-40 are assigned.

As illustrated in Table 2, an oligonucleotide chip for investigating point mutation of rpoB gene is devised including seven wild-type probes, seventeen RIF-resistance probes, and eight RIB-sensitive probes.

As a result, seventeen RIF-resistance probes of SEQ ID NOs 20-36 can discriminate the mutations of codon 511 (Leu >Pro), codon 513 (Gln >Pro), codon 516 (a: Asp >Val, b: Asp >Tyr, c: Asp >Glu), codon 518 (Asn >His), codon 521 (Leu >Met), codon 522 (Ser >Leu), codon 526 (a: His )Tyr, b: His )Asp, c: His )Arg, d: His )Asn, e: His )Ala, f: His >Cys, g: His )Gln, h: His >Gly), and codon 531 (Ser >Leu).

Eight RIB-sensitive probes of SEQ ID NOs 22, 23 and 31-36 among seventeen RIF-resistance probes can discriminate the mutations of codon 516 (a: Asp >Val, b: Asp >Tyr, c: Asp >Glu), and codon 526 (d: His >Asn, e: His >Ala, f: His >Cys, g: His >Gln, h: His >Gly).

Strains having mutation type 516a and 516b have shown conflict with respect to RIB-susceptibility as indicated in many reports (James M. Musser, "Antimicrobial Agent Resistance in Mycobacteria: Molecular Genetic Insights", Clinical Microbiology Review, Vol.8, No. 4, pp. 496-514, 1995). The current format of the oligonucleotide chip includes 516a and 516b probes in RIB-sensitive probes.

Probes Mc1, Mc2, and Mc5 of SEQ ID NOs 1-3 devised in order to discriminate Mycobacterium species from other bacteria are mixed and affixed as a single spot on an oligonucleotide chip. From the positive reaction on the probes, Mc1 can detect M. tuberculosis, M. africanum, M. asiaticum, M. bovis (2), M. gastri, M. avium (2), M. celatium (2), M. genavense, M. gordonae, M. haemophilum, M. interjectum, M. intermedium, M. intracellulare, M. kansasii, M. leprae, M. malmoense, M. scrofulaceum, M. szulgai, M. xenopi, M. terrae, M. triviale, M. nonchromogenicum, M. aurum, M. chitae, M. peregrinum, M. phlei, M. smegmatis, M. thermoresistibile, and M. vaccae, Mc2 can detect pathogenic or non-pathogenic but clinically important M. marium, M. shimoidei, M. ulcerans, M. abscessus, and M. chelonae, and Mc5 can detect M. fortuitum. Also, probe Mex of SEQ ID NO 4 detecting Corynebacterium diphtheriae which is not mycobactria but belongs to similar but different genus as a contrast group, is added in order to compare hybridization signals with each other, to thereby enable a more accurate determination on whether or not the strain under examination belongs to Mycobacteria.

Eight Mycobacterial species identification probes of SEQ ID NO 5 through 12 including partial sequences of Mycobacterial species-specific rpoB gene have been added in order to perform Mycobacterial species identification in more detail, in addition to probes which can discriminate Mycobacteria from other similar bacteria. In the case of the Mycobacterium species identification probes, the Ms1 probe can detect M. tuberculosis, M. africanum, M. bovis, and M. gastri. The other probes have been devised to detect M. asiaticum (Ms2), M. simiae (Ms2), M. aurum (Ms2), M. senegalence (Ms2), M. shimoidei (Ms2), M. neoaurum (Ms2), M. terrae (Ms2), M. gordonae (Ms2), M. haemophilum (Ms2), M. szulgai (Ms2), M. intracellulare (Ms2), M. kansasii (Ms2), M. scrofulaceum (Ms2), M. fortuitum (Ms2), M. fallax (Ms2), M. flavescens (Ms2), M. peregrinum (Ms2), M. phlei (Ms2), M. vaccae (Ms2), M. marinumv (Ms2), M. ulcerans (Ms2), M. chitae (Ms2), M. genevense (Ms2), M. smegmatis (Ms2), M. intermedium (Ms3), M. malmonense (Ms3), M. nonchromogenicum (Ms3), M. leprae (Ms3), M. avium (Ms4), M. triviale (Ms4), M. celatium (Ms4), M. interjectum (Ms5), M. xenopi (Ms6), M. chelonae (Ms7), M. abscessus (Ms7), and M. thermoresistibile (Ms8) which are pathogenic or non-pathogenic but clinically significant and may need to be identified.

Additional species identification probes, MTAB and MF, have been devised to specifically detect M. tuberculosis (MTAB), M. africanum (MTAB), M. bovis (MTAB), M. bovis BCG (MTAB), M. intracelluare (MTAB), and M. kansasii (MTAB), M. fortuitum (MF), and M. flavescens (MF).

### Embodiment 1-3: Fabrication of oligonucleotide chip

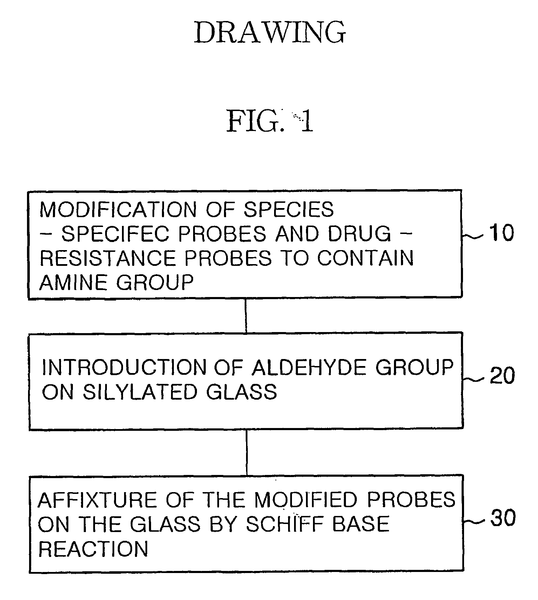

FIG. 1 is a flowchart view illustrating a process of fabricating an oligonucleotide chip for Mycobacterium DNA identification according to the present invention. As shown in FIG. 1, the oligonucleotide chip fabrication process includes: the first step (10) of modifying each of the Mycobacterium complex probes, Mycobacterium species identification probes having Mycobacterial species-specific DNA sequences of Mycobacterium rpoB gene, Mycobacterium drug-resistance detection probes including one or more modified codons of the Mycobacterium rpoB gene, and contrast group probes including wild-type sequences corresponding to each drug-resistance detection probes, to contain an amine group at the 5' terminal; the second step (20) of introducing an aldehyde group on silylated slide glass; and the third step (30) of affixing the modified probe on the slide glass by Schiff base reaction, to thereby fabricate an oligonucleotide chip.

More specifically, a particular DNA probe (see Table 2) was dissolved in 3×SSC to a concentration of 200pmol/µℓ. The DNA probe solution of 0.1-0.2µℓ was spotted on an aldehyde-derivatized slide glass (CEL Associates, Inc., MA, U.S.A), and reacted in a humidified incubator for 4 hours at 37°C. Then the slide glass was treated with 0.2% sodium dodecyl sulfate (SDS) solution for one minute, distilled water for one minute, and then with NaBH₄ solution (O.lg NaBH₄, 30ml PBS, and 10ml EtOH) for five minutes. Finally, the slide glass was washed for one minute in distilled water and air-dried, and kept in a dark room at room temperature until it is used.

### Embodiment 1-4: Polymerase chain reaction (PCR)

PCR has been performed in a total volume of 50µℓ with 10mM Tris-HCl (pH 8.3), 50mM KCl, 1.5mM MgCl₂, 1U Taq polymerase, 50pmol biotin-TR8 and TR9 primers (see Table 2). Further, 1mM dATP, dGTP, and dCTP of 2µℓ each, dTTP of 1.5µℓ, and 1mM biotin-dUTP of 0.5µℓ were added to randomly incorporate fluorescence labeling in the resulting target DNA. Table 3 illustrates the composition of PCR mixture.

**[Table 3]**

| PCR REACTION MIXTURE COMPOSITION | AMOUNT *(*µ*l)* |
|---|---|
| 10 × PCR buffer (MgCl₂,-free) | 5 |
| MgCl₂ (25 mM) | 3 |
| dNTPs (1 mM each) | 2 (dT 1.5) |
| Biotin-dUTP (1 mM) | 0.5 |
| Primer 1 : Bio-TR 8 (50 pmol/µl) | 2 |
| Primer 2 : TR 9 (50 pmol/µl) | 2 |
| Taq polymerase (5 unit/µl) | 0.2 |
| Template DNA | 1 |

The PCR was performed for 35 cycles under the following conditions.
- predenaturation: at 94°C, for 5 minutes
- denaturation: at 94°C, for 30 seconds
- primer annealing: at 63°C, for 30 seconds
- polymerization: at 72°C, for 45 seconds
- last extension: at 72°C, for 5 minutes

The PCR products produced through the above process are 157bp including 81bop core region, and has been identified by electrophoresis on a 2% agarose gel.

### Embodiment 1-5: Hybridization and Scanning

A target DNA (157bp) of 20µℓ amplified by PCR was mixed with DNase buffer of 20µℓ and DNase I (0.1U/µℓ) of 1µℓ, and treated at 0°C for 5 minutes. The resulting target DNA was thermally treated at 99°C for 10 minutes to inactivate the DNase I. The mixture was then treated with 3N NaOH of 4µℓ at 25 °C for 5 minutes, and then treated at 0°C for 5 minutes with Tris-HCl (pH 7.2) of 2µℓ and 3N HCl of 4µℓ. To the above mixture, 12 x SSPE (saline-sodium phosphate-EDTA buffer) of 50µℓ and 10% SDS of 0.5µℓ were added, and then transferred on to an oligonucleotide chip for hybridization at 40°C for 3 hours.

After hybridization, the oligonucleotide chip was washed with 2 x SSPE and 0.03% SDS at 25°C for 3 minutes, with 1 x SSPE at 25 °C for 5 minutes, and then with 0.2 x SSPE at 25 °C for 5 minutes to remove unreacted DNA. The oligonucleotide chip was dried at 25 °C, and a mixture of 3 x SSPE of 49µℓ and streptavidin-R-phycoerythrin of 1µℓ was added, then treated at 25°C for 10 minutes. The stained oligonucleotide chip was washed twice with 1 x SSPE for 1 minute, air-dried, and scanned with a laser fluorescence scanner (GMS 418 array scanner, TaKaRa, Japan).

The wild-type (wt) probes consist of seven 17mer probes devised from rpoB gene sequence corresponding to codons 507-533, including the mutation site in middle (positions 8-12) and have maximum sequence overlaps between the wild-type probes. The present invention has been devised to accurately detect frequent point mutations by comparing the signal intensities of the rifampin-resistance probes with those of the corresponding wild-type probes, and simultaneously predict all point mutations found at 507-533 codons.

Each mutant probe is designed to have the most frequent mutation site located in the middle position (8-12 positions) of the probe as in the wild-type probes, to thereby enhance detection sensitivitiy and enable more accurate detection by comparing the signal intensities with wild-type probes. The hybridization efficiency was increased by attaching a T₁₀ spacer on each probe, which is confirmed experimentally. It is considered that the T₁₀ spacer reduces steric hindrance and hence increases the hybridization efficiency of the target DNA and the probes.

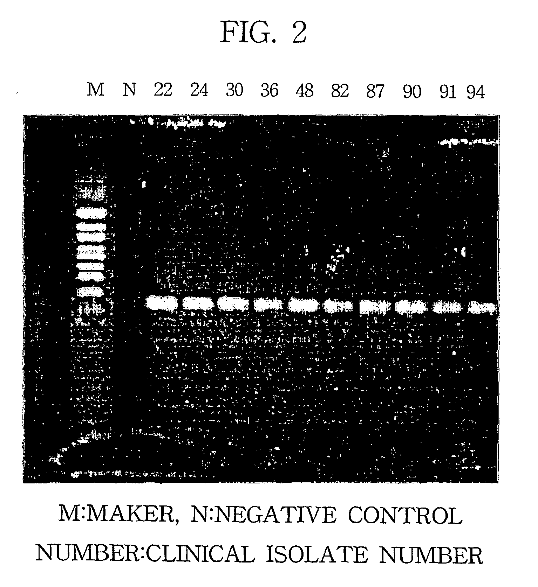

FIG. 2 is a pictorial view showing the result in which rpoB gene fragments (157bp) of ten clinical isolates are amplified using biotin-TR8 and TR9 primers and then undergone an electrophoresis in 2% agarose gel, according to the present invention. The ten amplified rpoB gene fragments (157bp) were digested with DNase I, and then hybridized with the oligonucleotide chip. After hybridization under the optimal conditions, that is, at 40°C for 3 hours, the oligonucleotide chip was stained with streptavidin-R-phycoerythrin for subsequent fluorescence scanning at 578nm. The resulting image can be analyzed for drug-resistance by comparing the fluorescent intensities of the wild-type (wt) probes with those of the mutation (mt) probes relatively. Mutant probes and wild-type probes have identical sequence except for a few base difference in the middle of the sequence. The target DNA of a mutant will show stronger signal on the corresponding mutant probe than its wild-type probe. The difference in hybridization intensity is visually recognized for determination. The signal intensity can also be arithmetically valued for more accurate comparison by using an appropriate software.

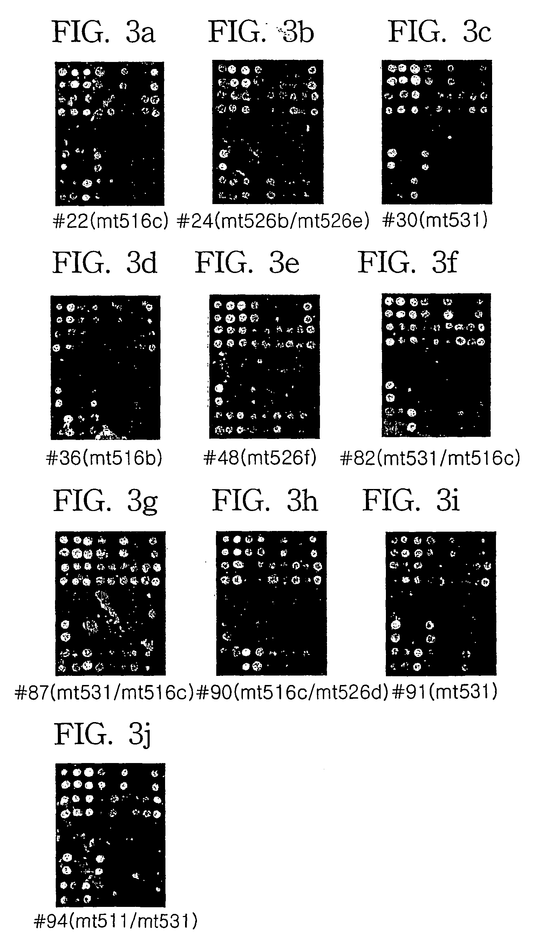

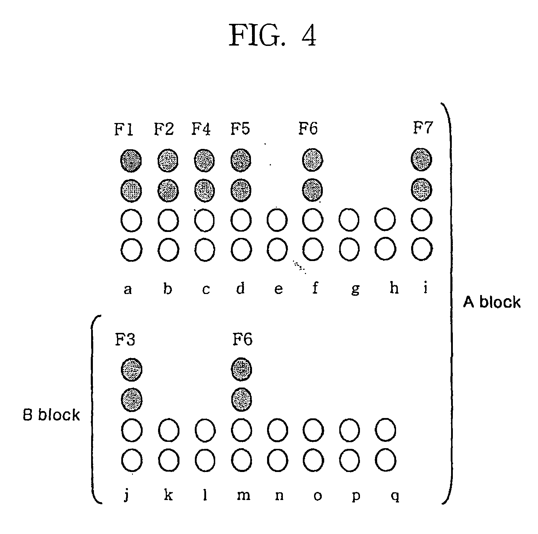

FIGs. 3A through 3J are pictorial views showing fluorescent images obtained by amplified clinical samples containing biotin-dUTP using an oligonucleotide chip, according to the present invention. FIG. 4 is a view showing the positions of the probes in the FIGs. 3A through 3J views, in which each probe is established into separate blocks to facilitate signal comparison and double spots are placed for each probe to increase analysis fidelity.

In FIG. 3A, the signals of the F1, F2, F4, F5, F6 and F7 which are the wild-type probes are stronger than those of the mutant probes, but the signal of the 516c mutant probe is stronger than those of the corresponding wild-type probe F3 and the mutant probes 516a and 516b. Therefore, the specimen was determined as a 516c mutant. In FIGs. 3B through 3J, the specimens were identified in the same manner as that of FIG. 3A.

As a result, as shown in FIGs. 3A through 3J, each clinical isolate was identified to contain the following RIF-resistance mutation(s): #22 (mt516c), #24 (mt516b/526e), #30 (mt531), #36 (mt516b), #48 (mt526f), #82 (mt531/516c), #87 (mt531/516c), #90 (mt516c/526d), #91 (mt531), and #94 (mt511/531).

Also, the clinical isolate revealing RIB-susceptibility among them were #22 (mt516c), #24 (mt526b/526e), #36 (mt516b), #48 (mt526f), #82 (mt531/516c), #87 (mt531/516c), and #90 (mt516c/526d). When the above result was compared with that of drug-susceptibility test result or DNA sequencing, a discrepancy was found at #24. It showed RIB-resistance in drug-susceptibility test but was determined as RIB-sensitive 526e mutant by sequencing. The oligonucleotide chip result showed signals on both RIB-resistant mt 526b and RIB-sensitive mt 526e. Based on these results, the sample is considered to contain two types of mutations or two different strains producing mixed results.

L. B. Heifets has reported that strains having mutations of 511 (Leu>Pro), 516 (Asp>Tyr), 516 (Asp>Val), and 522 (Ser>Leu) are classified as low-level resistance strains with rifabutin MIC≤ 5µg/ml (Heifets L. B., Antituberculosis drugs: antimicrobial activity in vitro, pp. 14-57 in L. B. Heifets (ed.), Drug susceptibility in the chemotherapy of mycobacterial infections, 1st ed. CRC Press, Boca Raton, Fla, 1991). For these cases, the drug-susceptibility test is necessary in addition to the oligonucleotide chip experiment.

In the cases of codon 526 mutants, it has been clearly determined that His>Gln, Gly, Asn, Ala, or Cys, are RIB-susceptibility whereas His>Tyr, Pro, Arg, or Asp are RIB-resistance, therefore, oligonucleotide chip experiment alone can discriminate RIB-susceptibility.

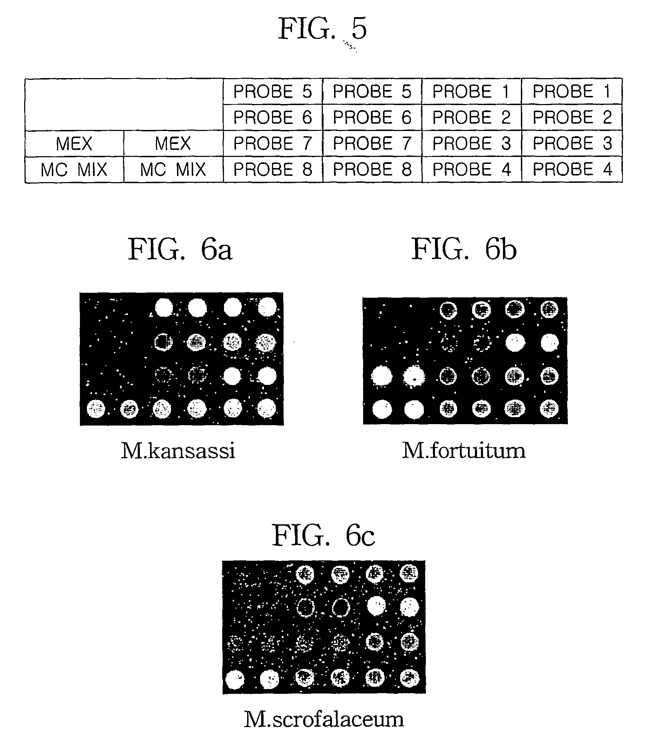

FIG. 5 is a diagram showing an array format of the oligonucleotide chip using probes Ms1-Ms8 designed for species identification. FIGs. 6A through 6C are pictorial views showing the experimental results of Mycobacterium species identification using the oligonucleotide chip of FIG. 5. In the case of Mycobacterium kansasii, Mycobacterium fortuitum, and Mycobacterium scrofulaseum, the signal of MCmix probe indicating mycobacteria appears more strongly than that of the control Mex probe, and the signal of number 2 probe is stronger than those of numbers 1, and 3-8 probes, among the species identification probes. Likewise, clinically significant 33 kinds of Mycobacteria can be identified. In the case of Mycobaterium tuberculosis, the signals of the Mcmix probe and number 1 probe turn on, MOTT except for Mycobacterium gastri can be confirmed by signals on the Mcmix probe and the number 2 through number 8 probes.

In the following embodiment 2, the materials used were: sixteen different species of Mycobacterial genomic DNA have been provided from the Jeju University (Jeju, Korea). DNA probes and primers were custom-synthesized from Bionics (Seoul, Korea). All other reagents were first-grade.

### Embodiment 2-1: Fabrication of oligonucleotide probe

Additional probes detecting mutations of the rpoB gene of rifampin-resistance Mycobacterium tuberculosis can be used with above-mentioned drug-resistance probes. Base sequences of three additional probes and their corresponding wild-type probes are shown in Table 4.

**[Table 4]**

| PROBE | BASE SEQUENCE | DRUG-SUSCEPTIBILITY | IDENTIFICATION |
|---|---|---|---|
| Mt509 | 5'amine-t10-ggcaccagacagctgag-3' | RFP-R, RBU-S | 509 MUTANT |
| Mt533 | 5'amine-t10-tgtcggcgccggggccc-3' | RFP-R, RBU-R | 533 MUTANT |
| Mt524 | 5'amine-t10-tgtcggggttaacccac-3' | RFP-R, RBU-R | 524 MUTANT |
| Wt509 | 5'amine-t10-ggcaccagccagctgag-3' | RFP-S, RBU-S | 509 WILD TYPE |
| Wt533 | 5'amine-t10-tgtcggcgctggggccc-3' | RFP-S. RBU-S | 533 WILD TYPE |
| Wt524 | 5'amine-t10-tgtcggggttgacccac-3' | RFP-S, RBU-S | 524 WILD TYPE |

By comparing fluorescent intensities of probes, mutations such as codon 509(Ser)-AGA(Arg), codon 533 CTG(Leu)-CCG(Pro), codon 524 TTG(Leu)→TTA(Leu) can be detected. In Table 4, RFP represents rifampin, RBU represents rifabutin, R represents resistance, and S represents susceptibility.

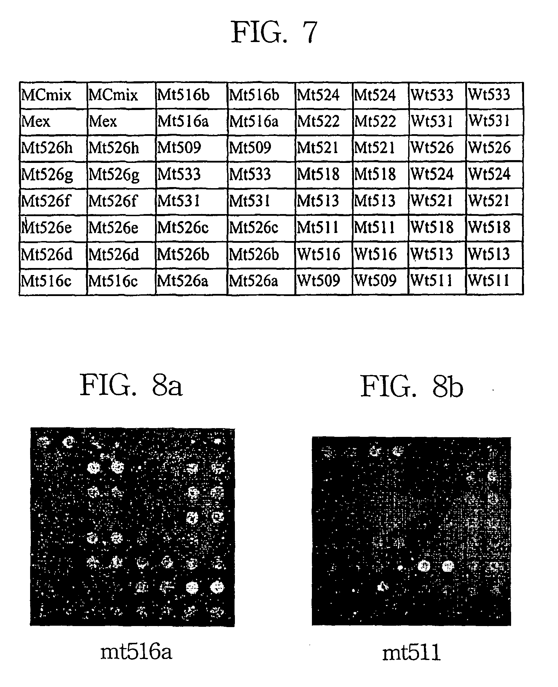

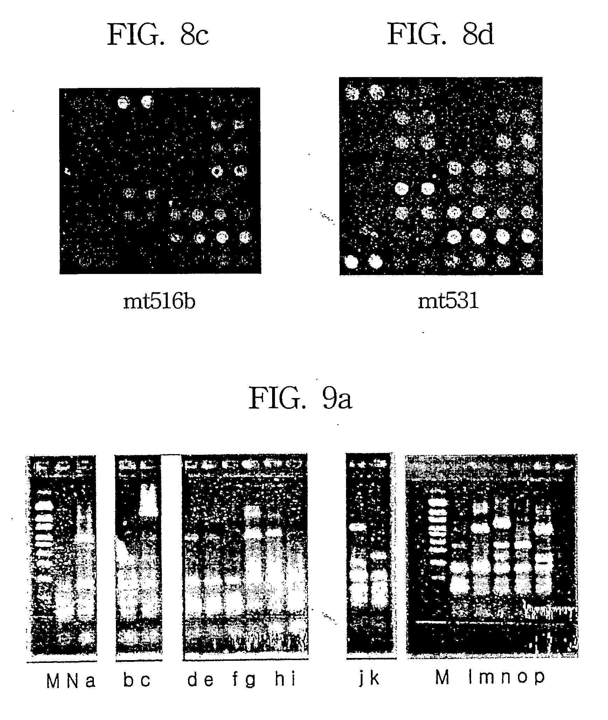

FIG. 7 is a diagram showing the array format of an oligonucleotide chip containing additional RIF-resistant probes. FIGs. 8A through 8D are pictorial views showing the diagnostic result regarding rifampin-resistance and rifabutin-sensitivity of clinical isolates using the oligonucleotide chip of FIG. 7. The clinical isolates used in the experiment were supplied from the Asan Medical Center (Seoul, Korea). Mutations in the samples were identified in the same manner as that of the embodiment 1 as #1 (516a mutant), #2 (511 mutant), #3 (516b mutant), and #4 (531 mutant), as shown in FIGs. 8A-8D. All the samples were Mycobacteria strains as judged by comparing Mcmix probe with Mex probe. The result of DNA sequencing agreed with the oligonucleotide chip results in all cases.

### Embodiment 2-2: Fabrication of primer

The inventors used biotin-TR8 and TR9 primer set in the prior application, but has devised a new primer set, biotin-DGR8, DGR9, that can amplify Mycobacterium tuberculosis and MOTT from uncultured specimens such as sputum, with an increased efficiency compared to biotin-TR8/TR9.

The new primer set, biotin-DGR8, DGR9, has been devised by mixing partial base sequences of rpoB genes of forty-three kinds, that is, M. africanum, M. asiaticum, M. avium, M. bovis, M. bovis BCG strain French 1173P2, M. celatum strain ATCC51131, M. celatum strain ATCC51130, M. gastri, M. genavense, M. gordonae, M haemophilum, M. interjectum, M. intermedium, M. intracellulare, M. kansasii, M. leprae, M. malmoense, M. marinum, M. avium subsp. Paratuberculosis, M. scrofulaceum, M. shimoidei, M. simiae, M. szulgai, M. ulcerans, M. xenopi, M. terrae, M. triviale, M. nonchromogenicum, M. abscessus, M. aurum, M. chelonae, M. chitae, M. fallax, M. flavescens, M. fortuitum strain ATCC6841, M. fortuitum strain ATCC49403, M. neoaurum, M. peregrinum, M. phlei, M. senegalense, M. smegmatis, M. thermoresistibile, and M. vaccae, in addition to the Mycobacterium tuberculosis.

The partial base sequences of the rpoB gene used to devise biotin-DGR8 and DGR9 primers are illustrated as the SEQ ID NOs 49-92 in Table 5.

**[Table 5]**

| | Species | DGR9 | DGR8 |
|---|---|---|---|
| 1 | *M. tuberculosis* | tc gccgcgatca aggagt | gga ggtccgcgac gtgca |
| 2 | *M. africanum* | tc gccgcgatca aggagt | gga ggtccgcgac gtgca |
| 3 | *M. asiaticum* | tt gccgcgatca aggagt | gga agtgcgtgac gtgca |
| 4 | *Mycobacterium avium* | tg gcggcgatca aggagt | gga ggtccgcgac gtgca |
| 5 | *Mycobacterium bovis* | tc gccgcgatca aggagt | gga ggtccgcgac gtgca |
| 6 | *Mycobacterium bovis* BCG strain French 1173P2 | tc gccgcgatca aggagt | gga ggtccgcgac gtgca |
| 7 | *Mycobacterium celatum* strain ATCC51131 | tg gcggcgatca aggagt | ega ggtgcgcgac gtgca |
| 8 | *Mycobacterium celatum* strain ATCC51130 | tg gcggccatca aggagt | gga ggtccgcgac gtgca |
| 9 | *Mycobacterium gastri* | tc gccgccatta aggagt | gga ggtccgcgac gtgca |
| 10 | *Mycobacterium genavense* | tg gcggcgatca aggagt | cga ggtccgcgac gtgca |
| 11 | *Mycobacterium gordonae* | tc gccgcgatca aggagt | gga agtacgtgac gtgca |
| 12 | *Mycobacterium haemophilum* | tc gccgcgatca aggagt | gga agtacgtgac gtgca |
| 13 | *Mycobacterium interjectum* | tc gccgcgatca aggagt | cga ggtccgcgac gtgca |
| 14 | *Mycobacterium intermedium* | tc gccgcgatca aggagt | gga agtccgtgac gtgca |
| 15 | *Mycobacterium intracellulare* | tc gccgcgatca aggagt | gga ggtccgtgac gtcca |
| 16 | *Mycobacterium kansasii* | tc gccgcgatca aggagt | gga ggtccgtgac gtcca |
| 17 | *Mycobacterium leprae* | tc gccgctatca aggaat | aga ggtccgtgac gtgca |
| 18 | *Mycobacterium malmoense* | tc gccgcgatca aggagt | gga ggtccgtgac gtgca |
| 19 | *Mycobacterium marinum* | tt gcggcgatca aggagt | gga agttcgtgac gtgca |
| 20 | *Mycobacterium avium* subsp. Paratuberculosis | tg gcggcgatca aggagt | gga ggtccgcgac gtgca |
| 21 | *Mycobacterium scrofulaceum* | tg gcggcgatca aggagt | tga ggtccgcgac gtgca |
| 22 | *Mycobacterium shimoidei* | tt gccgcgatca aggagt | gga agttcgtgac gtgca |
| 23 | *Mycobacterium simiae* | tg gcggcgatca aggagt | tga ggtccgcgac gtgca |
| 24 | *Mycobacterium szulgai* | tc gccgcgatca aggagt | gga ggtccgtgac gtgca |
| 25 | *Mycobacterium ulcerans* | tt gccgcgatca aggagt | gga agttcgtgac gtgca |
| 26 | *Mycobacterium xenopi* | tg gccgcgatca aggagt | gga ggtccgtgac gtgca |
| 27 | *Mycobacterium terrae* | tc gccgcgatca aggagt | tga ggtccgtgac gtgca |
| 28 | *Mycobacterium triviale* | tc gccgcgatca aggagt | gga ggtccgcgac gtgca |
| 29 | *Mycobacterium nonchromogenicum* | tc gccgccatca aggaat | gga agttcgtgac gtgca |
| 30 | *Mycobacterium abscessus* | tg gcggcgatca aggagt | cga ggtccgcgac gtgca |
| 31 | *Mycobacterium aurum* | tt gccgcgatca aggagt | gga agtgcgtgac gtgca |
| 32 | *Mycobacterium chelonae* | tg gcggcgatca aggagt | tga ggtccgcgac gtgca |
| 33 | *Mycobacterium chitae* | tg gcggcgatca aggagt | cga ggttcgtgac gtgca |
| 34 | *Mycobacterium fallax* | tg gcggcgatca aggagt | gga ggtccgcgac gtgca |
| *35* | *Mycobacterium flavescens* | tg gcggcgatca aggagt | cga agtccgtgac gtgca |
| 36 | *Mycobacterium fortuitum* strain ATCC6841 | tg gcggegatca aggagt | tga ggtccgcgac gtcca |
| 37 | *Mycobacterium fortuitum* strain ATCC49403 | tg gcggcgatca aggagt | tga ggtccgcgac gtcca |
| 38 | *Mycobacterium neoaurum* | tg gcggcgatca aggagt | tga ggtccgcgac gtgca |
| 39 | *Mycobacterium peregrinum* | tg gcggcgatca aggagt | tga ggtccgcgac gtgca |
| 40 | *Mycobacterium phlei* | tg gcggcgatca aggagt | cga ggtccgcgac gtgca |
| 41 | *Mycobacterium senegalense* | tg gcggcgatca aggagt | tga ggtccgcgac gtgca |
| 42 | *Mycobacterium smegmatis* | tg gcggcgatca aggagt | cga ggtccgcgac gtgca |
| 43 | *Mycobacterium thermoresistibile* | tg gcggcgatca aggagt | cga ggtccgcgac gtcca |
| 44 | *Mycobacterium vaccae* | tg gcggcgatca aggaat | cga ggtccgcgac gtgca |

In Table 6, the base sequences of the biotin-DGR8 and DGR9 fabricated as described above are illustrated and compared with the primer biotin-TR8 and TR9 of the prior application.

**[Table 6]**

| PRIMER | BASE SEQUENCE |
|---|---|
| biotin-TR8 | 5' biotin t g c a c g t c g c g g a c c t c c 3' |
| TR9 | 5' t c g c c g c g a t c a a g g a g t 3' |
| biotin-DGR8 | 5' biotin t g S a c g t c R c g N a c Y t c 3' |
| DGR9 | 5' t B g c S g c B a t Y a a g g a R t 3' |

Reference) B: c, t, g
N: c, t, g, a
R: g, a
S:c,g
Y:c,t

### Embodiment 2-3: Polymerase chain reaction (PCR)

In order to find optimal conditions for PCR using the newly devised biotin-DGR8 and DGR9 primer set, reaction conditions were varied as follows:

### A. Temperature program

a. at 94°C for 5 minutes, at 94°C for 30 seconds, at 47°C for 30 seconds, and at 72°C for 45 seconds
b. at 94°C for 5 minutes, at 94°C for 30 seconds, at 63°C for 30 seconds, and at 72°C for 45 seconds
c. at 94°C for 5 minutes, at 94 °C for 30 seconds, at 64 °C for 30 seconds, and at 72 °C for 45 seconds
d. at 94°C for 5 minutes, at 94°C for 30 seconds, at 65°C for 30 seconds, and at 72°C for 45 seconds

The yield of PCR product using the biotin-DGR8 and biotin-DGR9 primers changed depending on the annealing temperature, as dertermined by a 2% agarose gel electrophoresis. At the annealing temperature of 64 or 65°C, maximum yield was obtained with less amount of side products of different size that appeared at 47 or 63°C.

### B. Reaction cycle

Using the biotin-DGR8 and biotin-DGR9 primers, the amplified products of the polymerase chain reaction performed at 64°C annealing temperature showed a single band with 35 cycles or 40 cycles as identified by a 2% agarose gel. The quantity of the amplified products was observed to be dependent on the reaction cycle, producing higher yield at 40 cycles. The most optimal PCR conditions were 64°C and 40 cycles.

### C. Primer concentration

Using various concentrations of biotin-DGR8 and DGR9 primers, 50pmol, 100pmol, 500pmol, and 1000pmol, the polymerase chain reaction was performed at an annealing temperature of 64°C for 40 cycles. It was observed that the yield increased within 50∼100pmol concentration range but decreased with over 100 pmol. Therefore, biotin-DGR8 and DGR9 primer concentrations of 50-100pmol are considered appropriate.

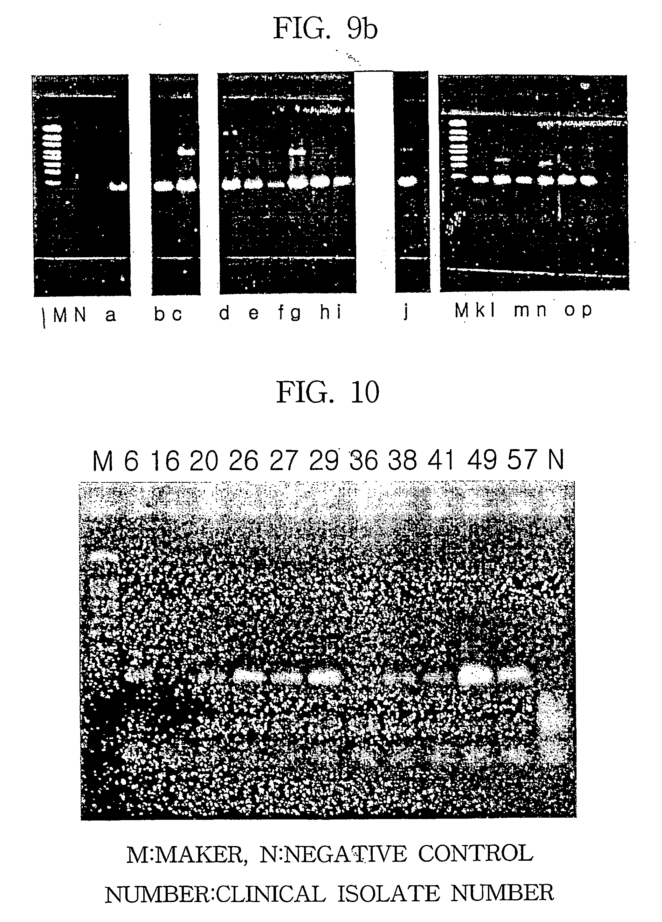

Using the optimal conditions determined experimentally as described above, sixteen different Mycobacterial species were PCR amplified. FIG. 9A is a pictorial view showing a 2% agarose gel image of PCR amplified products under the reaction conditions disclosed in the prior application of the inventors, that is, at 63°C and 35 cycles with biotin-TR8 and TR9 primers of 50pmol, and FIG. 9B is a pictorial view showing a 2% agarose gel image of PCR amplified products under the reaction conditions according to the present invention that is, at 64°C and 40 cycles with biotin-DGR8 and biotin-DGR9 primers of 100pmol.

As illustrated in FIGs. 9A and 9B, the primer set of the present invention, biotin-DGR8 and DGR9, showed more efficient amplification of the rpoB 157bp region in all sixteen Mycobacterial species compared to biotin-TR8 and TR9 primers. The 157bp amplified products can be used as the target DNA for oligonucleotide chip analysis.

### Embodiment 2-4: Drug-resistance diagnosis of sputum specimen

Eleven DNA samples directly extracted from sputum specimens of tuberculosis patients or suspected patients were obtained from the Department of Respiratory Diseases, Asan Medical Center (Seoul, Korea).

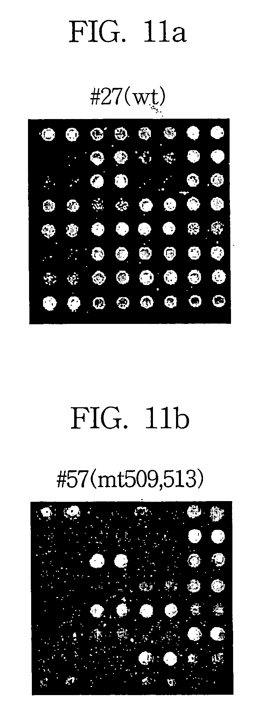

FIG. 10 is a pictorial view showing a 2% agarose gel image of PCR amplified products of sputum specimens under conditions as optimized in the embodiment 2-3 according to the present invention, i.e. 64°C and 40 cycles with biotin-DGR8 and DGR9 primers of 100pmol. As shown in FIG. 10, Mycobacterium tuberculosis was not detected from #16 and #36 specimens. Nine specimens except for the above #16 and #36 specimens were determined as Mycobacterium tuberculosis positive. Thus, diagnosis by oligonucleotide chip to detect drug-resistance was performed using the oligonucleotide chip of FIG 7. As a result, it was determined that eight specimens including #6, #20, #26, #27, #29, #38, #41, #49 were wild-type, that is, rifampin sensitive strains, and #57 was a resistant strain having mutations on codons 509 and 513. FIGs. 11A through 11B show the images obtained from oligonucleotide chip experiments: #27 (wild-type) #57 (509, 513 mutant), respectively. DNA sequencing result of the eight wild-type strains agreed with the oligonucleotide chip result and #57 was determined to be a rifampin-resistance strain by drug-susceptibility test.

### Embodiment 2-5: Random labeling incorporating Cyanine 5-dUTP

A new detection method has been devised to reduce assay time compared to the prior application method that uses streptavidin-R-phycoerythrin staining after PCR incorporating random biotin labeling. More specifically, PCR was performed with Cyanine 5-dUTP (NEN, U.S.A) instead of biotin-dUTP to incorporate Cyanine randomly to the amplified products, which does not require a separate staining procedure prior to scanning. Accordingly, an obvious visual result can be obtained speedily and simply. At the time of a polymerase chain reaction, a concentration of Cyanine 5-dUTP used equals to that of biotin-dUTP of the existing method, and it is preferable that DGR8 without biotin modification is used as a primer. The experimental conditions are the same as the existing method except that the staining process is omitted and that 633nm is used in fluorescence scanning

The pars which are not particularly described in the embodiments 2-1 through 2-5 are same as those of the embodiments 1-1 through 1-5, which will thus be omitted.

### Industrial Applicability

Finally, as described above, the present invention can perform Mycobacterial species identification and drug-resistance detection with only a portion of Mycobacterial rpoB gene (157bp), simultaneously, speedily and accurately in large quantity, which can provide useful information in tuberculosis treatment so that an appropriate drug remedy can be given to the patients. Thus, at an early stage of the Mycobacterium infection, a proper administration of antibiotic agents and other necessary treatment can be provided to patients infected with mycobacteria.

## Claims

1. A diagnosis kit for Mycobacterial species identification and drug-resistance detection comprising:
an oligonucleotide chip including species identification probes comprising SEQ ID NOs 5 through 12, a Mycobacterial rifampin resistance detection probe selected from SEQ ID NOs:20 through 36 and 41 through 46, and a control group probe comprised of wild-type sequences selected from SEQ ID NO: 13 through 19; and
a marker for detecting a hybridization of said oligonucleotide chip and a specimen.

2. The diagnosis kit for Mycobacterial species identification and drug-resistance detection of claim 1, wherein said drug-resistance detection probe further comprises a rifabutin susceptibility detection probe.

3. The diagnosis kit for Mycobacterial species identification and drug-resistance detection of claim 2, wherein said rifabutin susceptibility detection probe comprises modified codons 516 and 526 of the rpoB gene.

4. The diagnosis kit for Mycobacterial species identification and drug-resistance detection of claim 3, wherein said rifabutin susceptibility detection probe comprises probes of SEQ ID NOs 22, 23, and 31 through 36.

5. The diagnosis kit for Mycobacterial species identification and drug-resistance detection of claim 1, wherein said oligonucleotide chip is formed by a Schiff base reaction of each probe comprised of part of rpoB gens modified to contain an amine group and an aldehyde group induced on glass.

6. The diagnosis kit for Mycobacterial species identification and drug-resistance detection of claim 1, further comprising a means for amplifying the DNAs of the specimen.

7. The diagnosis kit for Mycobacterial species identification and drug-resistance detection of claim 6, wherein said means comprises biotin-TR8 and TR9 primers of SEQ ID NOs 37 and 38 which amplify rpoB gene fragments 157bp specifically.

8. The diagnosis kit for Mycobacterial species identification and drug-resistance detection of claim 6, wherein said means comprises biotin-DGR8 and DGR9 primers of SEQ ID NOs 47 and 48 which amplify rpoB gene fragments having 157bp specifically.

9. The diagnosis kit for Mycobacterial species identification and drug-resistance detection of claim 1, wherein said marker is a fluorescent material including said biotin-binding protein.

10. The diagnosis kit for Mycobacterial species identification and drug-resistance detection of claim 9, wherein said fluorescent material is streptavidin-R-phycoerythrin.

11. The diagnosis kit for Mycobacterial species identification and drug-resistance detection of claim 6, wherein said marker is Cynine 5-dUTP added in the polymerase chain reaction.

12. The diagnosis kit for Mycobacterial species identification and drug-resistance detection of claim 1, wherein said each probe comprises T₁₀ included at 5' as a spacer.

13. The diagnosis kit for Mycobacterial species identification and drug-resistance detection of claim 1, wherein said oligonucleotide chip further comprises a Mycobacterium complex probe which can detect whether a specimen is Mycobacterium.

14. The diagnosis kit for Mycobacterial species identification and drug-resistance detection of claim 13, wherein said tuberculosis bacteria group probe comprises probes of SEQ ID NOs 1 through 4.

## Patentansprüche

1. Diagnosekit zur Identifikation und Detektion der Wirkstoffresistenz einer mykobakteriellen Spezies, umfassend:
einen Oligonucleotidchip, der Speziesidentifikationssonden, umfassend SEQ ID NR. 5 bis 12, eine Sonde zur Detektion von Mykobakterien-Rifampicinresistenz, ausgewählt aus SEQ ID NR. 20 bis 36 und 41 bis 46, und eine Kontrollgruppensonde, bestehend aus Wildtypsequenzen, ausgewählt aus SEQ ID NR. 13 bis 19, umfasst; und
einen Marker zur Detektion einer Hybridisierung des Oligonucleotidchips und einer Probe.

2. Diagnosekit zur Identifikation und Detektion der Wirkstoffresistenz einer mykobakteriellen Spezies nach Anspruch 1, wobei die Sonde zur Detektion der Wirkstoffresistenz ferner eine Sonde zur Detektion der Rifabutinempfindlichkeit umfasst.

3. Diagnosekit zur Identifikation und Detektion der Wirkstoffresistenz einer mykobakteriellen Spezies nach Anspruch 2, wobei die Sonde zur Detektion der Rifabutinempfindlichkeit modifizierte Codone 516 und 526 des rpoB-Gens umfasst.

4. Diagnosekit zur Identifikation und Detektion der Wirkstoffresistenz einer mykobakteriellen Spezies nach Anspruch 3, wobei die Sonde zur Detektion der Rifabutinempfindlichkeit Sonden der SEQ ID NR. 22, 23 und 31 bis 36 umfasst.

5. Diagnosekit zur Identifikation und Detektion der Wirkstoffresistenz einer mykobakteriellen Spezies nach Anspruch 1, wobei der Oligonucleotidchip durch eine Schiff'sche Base-Reaktion jeder Sonde, die aus einem Teil von rpoB-Genen besteht, die so modifiziert sind, dass sie eine Amingruppe und eine Aldehydgruppe enthalten, die auf Glas induziert wird, gebildet wird.

6. Diagnosekit zur Identifikation und Detektion der Wirkstoffresistenz einer mykobakteriellen Spezies nach Anspruch 1, ferner umfassend ein Mittel für die Amplifikation der DNAs der Probe.

7. Diagnosekit zur Identifikation und Detektion der Wirkstoffresistenz einer mykobakteriellen Spezies nach Anspruch 6, wobei das Mittel Biotin-TR8- und -TR9-Primer der SEQ ID NR. 37 und 38 umfasst, die rpoB-Genfragmente mit 157bp spezifisch amplifizieren.

8. Diagnosekit zur Identifikation und Detektion der Wirkstoffresistenz einer mykobakteriellen Spezies nach Anspruch 6, wobei das Mittel Biotin-DGR8- und -DGR9-Primer der SEQ ID Nr. 47 und 48 umfasst, die rpoB-Genfragmente mit 157bp spezifisch amplifizieren.

9. Diagnosekit zur Identifikation und Detektion der Wirkstoffresistenz einer mykobakteriellen Spezies nach Anspruch 1, wobei der Marker ein fluoreszierender Stoff ist, der das Biotin-Bindeprotein einschließt.

10. Diagnosekit zur Identifikation und Detektion der Wirkstoffresistenz einer mykobakteriellen Spezies nach Anspruch 9, wobei der fluoreszierende Stoff Streptavidin-R-phycoerythrin ist.

11. Diagnosekit zur Identifikation und Detektion der Wirkstoffresistenz einer mykobakteriellen Spezies nach Anspruch 6, wobei der Marker Cynine-5-Dutp ist, der bei der Polymerase-Kettenreaktion zugegeben wird.

12. Diagnosekit zur Identifikation und Detektion der Wirkstoffresistenz einer mykobakteriellen Spezies nach Anspruch 1, wobei jede Sonde T₁₀, enthalten an 5' als ein Spacer, umfasst.

13. Diagnosekit zur Identifikation und Detektion der Wirkstoffresistenz einer mykobakteriellen Spezies nach Anspruch 1, wobei der Oligonucleotidchip ferner eine Mykobakteriumkomplexsonde umfasst, die detektieren kann, ob eine Probe ein Mykobakterium ist.

14. Diagnosekit zur Identifikation und Detektion der Wirkstoffresistenz einer mykobakteriellen Spezies nach Anspruch 13, wobei die Sonde der Tuberkulosebakteriengruppe Sonden der SEQ ID NR. 1 bis 4 umfasst.

## Revendications

1. Kit de diagnostic pour l'identification d'espèces mycobactériennes et la détection de la résistance à un médicament, comprenant :
une puce d'oligonucléotide comprenant des sondes d'identification d'espèce comprenant SEQ ID N° : 5 à 12, une sonde de détection de la résistance à la rifampine choisie dans SEQ ID N° : 20 à 36 et 41 à 46 et une sonde de groupe témoin comprenant des séquences de type sauvage de SEQ ID N° : 13 à 19 ; et
un marqueur pour détecter une hybridation de ladite puce d'oligonucléotide et d'un échantillon.

2. Kit de diagnostic pour l'identification d'espèces mycobactérienne et de détection de la résistance à un médicament selon la revendication 1, dans lequel ladite sonde de détection de la résistance à un médicament comprend en outre une sonde de détection de la sensibilité à la rifabutine.

3. Kit de diagnostic pour l'identification d'espèces mycobactériennes et de détection de la résistance à un médicament selon la revendication 2, dans lequel ladite sonde de détection de la sensibilité à la rifabutine comprend les codons 516 et 526 modifiés du gène rpoB.

4. Kit de diagnostic pour l'identification d'espèces mycobactériennes et de détection de la résistance à un médicament selon la revendication 3, dans lequel ladite sonde de détection de la sensibilité à la rifabutine comprend les sondes de SEQ ID N° 22, 23, 31 à 36.

5. Kit de diagnostic pour l'identification d'espèces mycobactériennes et de détection de la résistance à un médicament selon la revendication 1, dans lequel ladite puce d'oligonucléotide est formée par la réaction d'une base de Schiff de chaque sonde constituée d'une partie des gènes rpoB modifiée de façon à contenir un groupe amine et un groupe aldéhyde induits sur verre.

6. Kit de diagnostic pour l'identification d'espèces mycobactériennes et de détection de la résistance à un médicament selon la revendication 1, comprenant en outre un moyen d'amplification des ADN de l'échantillon.

7. Kit de diagnostic pour l'identification d'espèces mycobactériennes et de détection de la résistance à un médicament selon la revendication 6, dans lequel ledit moyen comprend des amorces de biotine-TR8 et TR9 de SEQ ID N° 37 et 38 qui amplifient les fragments de gène rpoB de 157 pb spécifiquement.

8. Kit de diagnostic pour l'identification d'espèces mycobactériennes et de détection de la résistance à un médicament selon la revendication 6, dans lequel ledit moyen comprend des amorces de biotine-DGR8 et DGR9 de SEQ ID N° 47 et 48 qui amplifient des fragments de gène rpoB ayant 157 pb spécifiquement.

9. Kit de diagnostic pour l'identification d'espèces mycobactériennes et de détection de la résistance à un médicament selon la revendication 1, dans lequel ledit marqueur est un matériau fluorescent comprenant ladite protéine se liant à la biotine.

10. Kit de diagnostic pour l'identification d'espèces mycobactériennes et de détection de la résistance à un médicament selon la revendication 9, dans lequel ledit matériau fluorescent est la streptavidine-R-phycoérythrine.

11. Kit de diagnostic pour l'identification d'espèces mycobactériennes et de détection de la résistance à un médicament selon la revendication 6, dans lequel ledit marqueur est la cynine 5-dUTP ajoutée à la réaction en chaîne polymérase.

12. Kit de diagnostic pour l'identification d'espèces mycobactériennes et de détection de la résistance à un médicament selon la revendication 1, dans lequel ladite sonde respective comprend T₁₀ inclus au niveau 5' comme segment d'espacement.

13. Kit de diagnostic pour l'identification d'espèces mycobactériennes et de détection de la résistance à un médicament selon la revendication 1, dans lequel ladite puce d'oligonucléotide comprend en outre une sonde complexe de mycobactérie qui peut détecter si un échantillon est une mycobactérie.

14. Kit de diagnostic pour l'identification d'espèces mycobactériennes et de détection de la résistance à un médicament selon la revendication 13, dans lequel ladite sonde du groupe de bactéries de la tuberculose comprend les sondes de SEQ ID N° : 1 à 4.


### Sequence Listing

<110> biomedlab
<120> diagnosis kit for Mycobacterium species identification and drug-resistance detection and manufaturing method thereof
<130> SGG1601PCT
<150> KR10-2000-0029369
   <151> 2000-05-30
<160> 92
<170> KopatentIn 1.71
<210> 1
   <211> 17
   <212> DNA
   <213> Mycobacterium tuberculosis
<400> 1
   tcttcggcac cagccag 17
<210> 2
   <211> 17
   <212> DNA
   <213> Mycobacterium marinum
<400> 2
   tcttcggaac cagccag 17
<210> 3
   <211> 17
   <212> DNA
   <213> Mycobacterium fortuitum
<400> 3
   tcttcggaac gtcgcag 17
<210> 4
   <211> 17
   <212> DNA
   <213> Corynebacterium diphtheriae
<400> 4
   tcttcggaac ctcgcag 17
<210> 5
   <211> 19
   <212> DNA
   <213> Mycobacterium tuberculosis,M.africanum,M.bovis,M.gastri,MTB complex
<400> 5
   ggtctgtcac gtgagcgtg 19
<210> 6
   <211> 19
   <212> DNA
   <213> Mycobacterium asiaticum,M.simiae,M.aurum,M.senegalense,M.shimoidei,M.teN.neoaurum
<400> 6
   ggtctgtccc gtgagcgtg 19
<210> 7
   <211> 19
   <212> DNA
   <213> Mycobacterium intermedium,M.leprae,M.malmoense,M.nonchromogenicum
<400> 7
   ggtctgtcgc gtgagcgtg 19
<210> 8
   <211> 19
   <212> DNA
   <213> Mycobarerium avium paratuberculosis,M.avium,M.triviale,M.celatium
<400> 8
   ggtctgtccc gggagcgtg 19
<210> 9
   <211> 19
   <212> DNA
   <213> Mycobacterium interjectum
<400> 9
   ggtctgtccc gcgagcgtg 19
<210> 10
   <211> 19
   <212> DNA
   <213> Mycobacterium xenopi
<400> 10
   ggtctgtcgc gcgagcgtg 19
<210> 11
   <211> 19
   <212> DNA
   <213> Mycobacterium chelonae, M. abscessus
<400> 11
   ggtctgaccc gtgaccgtg 19
<210> 12
   <211> 19
   <212> DNA
   <213> Mycobacterium thermoresistibile
<400> 12
   ggtctgagcc gggagcgtg 19
<210> 13
   <211> 17
   <212> DNA
   <213> Mycobacterium tuberculosis
<400> 13
   cagccagctg agccaat 17
<210> 14
   <211> 17
   <212> DNA
   <213> Mycobacterium tuberculosis
<400> 14
   gctgagccaa ttcatgg 17
<210> 15
   <211> 17
   <212> DNA
   <213> Mycobacterium tuberculosis
<400> 15
   attcatggac cagaaca 17
<210> 16
   <211> 17
   <212> DNA
   <213> Mycobacterium tuberculosis
<400> 16
   tggaccagaa caacccg 17
<210> 17
   <211> 17
   <212> DNA
   <213> Mycobacterium tuberculosis
<400> 17
   caacccgctg tcggggt 17
<210> 18
   <211> 17
   <212> DNA
   <213> Mycobacterium tuberculosis
<400> 18
   ggttgaccca caagcgc 17
<210> 19
   <211> 17
   <212> DNA
   <213> Mycobacterium tuberculosis
<400> 19
   ccgactgtcg gcgctgg 17
<210> 20
   <211> 17
   <212> DNA
   <213> Mycobacterium tuberculosis
<400> 20
   cagccagccg agccaat 17
<210> 21
   <211> 17
   <212> DNA
   <213> Mycobacterium tuberculosis
<400> 21
   gctgagccca ttcatgg 17
<210> 22
   <211> 17
   <212> DNA
   <213> Mycobacterium tuberculosis
<400> 22
   attcatggtc cagaaca 17
<210> 23
   <211> 17
   <212> DNA
   <213> Mycobacterium tuberculosis
<400> 23
   attcatgtac cagaaca 17
<210> 24
   <211> 17
   <212> DNA
   <213> Mycobacterium tuberculosis
<400> 24
   tggaccagca caacccg 17
<210> 25
   <211> 17
   <212> DNA
   <213> Mycobacterium tuberculosis
<400> 25
   caacccgatg tcggggt 17
<210> 26
   <211> 17
   <212> DNA
   <213> Mycobacterium tuberculosis
<400> 26
   caacccgctg ttggggt 17
<210> 27
   <211> 17
   <212> DNA
   <213> Mycobacterium tuberculosis
<400> 27
   ggttgaccta caagcgc 17
<210> 28
   <211> 17
   <212> DNA
   <213> Mycobacterium tuberculosis
<400> 28
   ggttgaccga caagcgc 17
<210> 29
   <211> 17
   <212> DNA
   <213> Mycobacterium tuberculosis
<400> 29
   ggttgacccg caagcgc 17
<210> 30
   <211> 17
   <212> DNA
   <213> Mycobacterium tuberculosis
<400> 30
   ccgactgttg gcgctgg 17
<210> 31
   <211> 17
   <212> DNA
   <213> Mycobacterium tuberculosis
<400> 31
   attcatggag cagaaca 17
<210> 32
   <211> 17
   <212> DNA
   <213> Mycobacterium tuberculosis
<400> 32
   ggttgaccaa caagcgc 17
<210> 33
   <211> 17
   <212> DNA
   <213> Mycobacterium tuberculosis
<400> 33
   ggttgaccgc caagcgc 17
<210> 34
   <211> 17
   <212> DNA
   <213> Mycobacterium tuberculosis
<400> 34
   ggttgacctg caagcgc 17
<210> 35
   <211> 17
   <212> DNA
   <213> Mycobacterium tuberculosis
<400> 35
   ggttgaccca gaagcgc 17
<210> 36
   <211> 17
   <212> DNA
   <213> Mycobacterium tuberculosis
<400> 36
   ggttgaccgg caagcgc 17
<210> 37
   <211> 18
   <212> DNA
   <213> Mycobacterium tuberculosis
<400> 37
   tgcacgtcgc ggacctcc 18
<210> 38
   <211> 18
   <212> DNA
   <213> Mycobacterium tuberculosis
<400> 38
   tcgccgcgat caaggagt 18
<210> 39
   <211> 18
   <212> DNA
   <213> Mycobacterium tuberculosis, M.africanum, M.bovis, M.bovisBCG, M.intrace, M.kansas
<400> 39
   gcagctgagc caattcat 18
<210> 40
   <211> 17
   <212> DNA
   <213> Mycobacterium fortuitum, M.flavescens
<400> 40
   cgacgtcgca gctgtcg 17
<210> 41
   <211> 17
   <212> DNA
   <213> Mycobacterium tuberculosis
<400> 41
   ggcaccagac agctgag 17
<210> 42
   <211> 17
   <212> DNA
   <213> Mycobacterium tuberculosis
<400> 42
   tgtcggcgcc ggggccc 17
<210> 43
   <211> 17
   <212> DNA
   <213> Mycobacterium tuberculosis
<400> 43
   tgtcggggtt aacccac 17
<210> 44
   <211> 17
   <212> DNA
   <213> Mycobacterium tuberculosis
<400> 44
   ggcaccagcc agctgag 17
<210> 45
   <211> 17
   <212> DNA
   <213> Mycobacterium tuberculosis
<400> 45
   tgtcggcgct ggggccc 17
<210> 46
   <211> 17
   <212> DNA
   <213> Mycobacterium tuberculosis
<400> 46
   tgtcggggtt gacccac 17
<210> 47
   <211> 17
   <212> DNA
   <213> Artificial Sequence
<220>
   <223> this sequence is combinated with the rpoB genes of 44 species belonged to Mycobaterium.
<400> 47
   tgsacgtcrc gnacytc 17
<210> 48
   <211> 18
   <212> DNA
   <213> Artificial Sequence
<220>
   <223> This sequence is combinated with the rpoB genes of 44 species belonged to Mycobacterium.
<400> 48
   tbgcsgcbat yaaggart 18
<210> 49
   <211> 306
   <212> DNA
   <213> Mycobacterium tuberculosis
<400> 49
   tgcgtacggt cggcgagctg atccaaaacc agatccgggt cggcatgtcg cggatggagc     60
   gggtggtccg ggagcggatg accacccagg acgtggaggc gatcacaccg cagacgttga    120
   tcaacatccg gccggtggtc gccgcgatca aggagttctt cggcaccagc cagctgagcc    180
   aattcatgga ccagaacaac ccgctgtcgg ggttgaccca caagcgccga ctgtcggcgc    240
   tggggcccgg cggtctgtca cgtgagcgtg ccgggctgga ggtccgcgac gtgcacccgt    300
   cgcact                                                               306
<210> 50
   <211> 306
   <212> DNA
   <213> Mycobacterium africanum
<400> 50
   tgcgtacggt cggcgagctg atccaaaacc agatccgggt cggcatgtcg cggatggagc     60
   gggtggtccg ggagcggatg accacccagg acgtggaggc gatcacaccg cagacgttga    120
   tcaacatccg gccggtggtc gccgcgatca aggagttctt cggcaccagc cagctgagcc    180
   aattcatgga ccagaacaac ccgctgtcgg ggttgaccca caagcgccga ctgtcggcgc    240
   tggggcccgg cggtctgtca cgtgagcgtg ccgggctgga ggtccgcgac gtgcacccgt    300
   cgcact                                                               306
<210> 51
   <221> 306
   <212> DNA
   <213> Mycobacterium asiaticum
<400> 51
<210> 52
   <211> 300
   <212> DNA
   <213> Mycobacterium avium
<400> 52
<210> 53
   <211> 306
   <212> DNA
   <213> Mycobacterium bovis
<400> 53
   tgcgtacggt cggcgagctg atccaaaacc agatccgggt cggcatgtcg cggatggagc      60
   gggtggtccg ggagcggatg accacccagg acgtggaggc gatcacaccg cagacgttga     120
   tcaacatccg gccggtggtc gccgcgatca aggagttctt cggcaccagc cagctgagcc     180
   aattcatgga ccagaacaac ccgctgtcgg ggttgaccca caagcgccga ctgtcggcgc     240
   tggggcccgg cggtctgtca cgtgagcgtg ccgggctgga ggtccgcgac gtgcacccgt     300
   cgcact                                                                306
<210> 54
   <211> 306
   <212> DNA
   <213> Mycobacterium bovis BCG strain French 1173P2
<400> 54
   tgcgtacggt cggcgagctg atccaaaacc agatccgggt cggcatgtcg cggatggagc      60
   gggtggtccg ggagcggatg accacccagg acgtggaggc gatcacaccg cagacgttga     120
   tcaacatccg gccggtggtc gccgcgatca aggagttctt cggcaccagc cagctgagcc     180
   aattcatgga ccagaacaac ccgctgtcgg ggttgaccca caagcgccga ctgtcggcgc     240
   tggggcccgg cggtctgtca cgtgagcgtg ccgggctgga ggtccgcgac gtgcacccgt     300
   cgcact                                                                306
<210> 55
   <211> 306
   <212> DNA
   <213> Mycobacterium celatum strain ATCC51131
<400> 55
   ttcgtaccgt cggtgagctg atccagaacc agatccgagt cggcatgtcc cgcatggagc 60
   gggtggtccg cgagcggatg accactcagg acgtcgaggc gatcacgccg cagaccctga 120
   tcaacatccg tcccgtcgtg gcggcgatca aggagttctt cggcaccagc cagctctcgc 180
   agttcatgga ccagaacaac ccgctgtccg ggctgaccca caagcggcgc ctgaacgcac 240
   tgggcccggg tggtctgtcc cgggagcggg cgggcctcga ggtgcgcgac gtgcacccga 300
   gtcact 306
<210> 56
   <211> 306
   <212> DNA
   <213> Mycobacterium celatum strain ATCC51130
<400> 56
   ttcgtaccgt cggtgagctg atccagaacc agatccgagt cggcatgtcc cgcatggagc 60
   gggtggtccg cgagcggatg accactcagg acgtcgaggc gatcacgccg cagaccctga 120
   tcaacatccg tcccgtcgtg gcggcgatca aggagttctt cggcaccagc cagctctcgc 180
   agttcatgga ccagaacaac ccgctgtccg ggctgaccca caagcggcgc ctgaacgcac 240
   tgggcccggg tggtctgtcc cgggagcggg cgggcctcga ggtgcgcgac gtgcacccga 300
   gtcact 306
<210> 57
   <211> 306
   <212> DNA
   <213> Mycobacterium gastri
<400> 57
   tgcgcacggt gggcgagctg atccagaacc agatccgggt cggcatgtcc aggatggagc 60
   gcgtcgtccg ggagcggatg accactcagg acgtcgaggc catcacgccg cagacgctga 120
   tcaacattcg cccggtggtc gccgccatta aggagttctt cggcaccagc cagctgtcgc 180
   agttcatgga ccagaacaac ccgctgtcgg gcctgaccca caagcgccgg ctttcggcgc 240
   tgggccccgg cggtctgtca cgtgagcgtg ccgggctgga ggtccgcgac gtgcacccgt 300
   cgcact 306
<210> 58
   <211> 306
   <212> DNA
   <213> Mycobacterium genavense
<400> 58
   tgcgcacggt gggcgatctg atccagaacc agatccgggt cggcatgtcg cggatggagc 60
   gggtggtccg tgagcggatg accactcagg acgtcgaggc catcacgccg cagaccctga 120
   tcaacatccg tccggttgtg gcggcgatca aggagttctt cggcaccagc cagctctcgc 180
   agttcatgga ccagaacaac ccgctgtcag gtctcaccca caagcgccgg ttgtcggcgc 240
   tggggccggg cggtctgtcc cgtgagcggg cgggcctcga ggtccgcgac gtgcacccgt 300
   cgcact 306
<210> 59
   <211> 306
   <212> DNA
   <213> Mycobacterium gordonae
<400> 59
   tgcgcaccgt gggcgagctg atccagaacc agatccgggt cggcatgtcc cggatggagc 60
   gcgtcgtgcg cgagcggatg accactcagg acgtcgaggc catcacgccg cagaccctga 120
   tcaacatccg gccggtcgtc gccgcgatca aggagttctt cggcaccagc cagctctcgc 180
   agttcatgga ccagaacaac ccgctgtcgg gtctgaccca caagcgtcgt ctgtcggcgc 240
   tggggccggg tggtctgtcc cgtgagcgtg cgggtctgga agtacgtgac gtgcacccgt 300
   cgcact 306
<210> 60
   <211> 306
   <212> DNA
   <213> Mycobacterium haemophilum
<400> 60
   tgcgcaccgt gggcgagctg atccagaacc agatccgggt cggcatgtcc cggatggagc 60
   gcgtcgtgcg cgagcggatg accactcagg acgtcgaggc catcacgccg cagaccctga 120
   tcaacatccg gccggtcgtc gccgcgatca aggagttctt cggcaccagc cagctctcgc 180
   agttcatgga ccagaacaac ccgctgtcgg gtctgaccca caagcgtcgt ctgtcggcgc 240
   tggggccggg tggtctgtcc cgtgagcgtg cgggtctgga agtacgtgac gtgcacccgt 300
   cgcact 306
<210> 61
   <211> 306
   <212> DNA
   <213> Mycobacterium interjectum
<400> 61
   tgcgcaccgt cggtgagctg atccagaacc agatccgggt cggcatgtcc aggatggagc 60
   gcgtcgtccg ggagcggatg accacccagg acgtcgaggc gatcacgccg cagacgctga 120
   tcaacatccg gccggtcgtc gccgcgatca aggagttctt cggcaccagc cagctgtcgc 180
   agttcatgga ccagaacaac ccgctgtcgg gcctcaccca caagcgccgc ctgtcggcgc 240
   tgggcccggg cggtctgtcc cgcgagcggg ccggcctcga ggtccgcgac gtgcacccga 300
   accact 306
<210> 62
   <211> 306
   <212> DNA
   <213> Mycobacterium intermedium
<400> 62
   tgcgtaccgt cggcgagctg atccagaacc agatccgggt cggcatgtcc cgcatggagc 60
   gcgttgtccg cgagcggatg accactcagg acgtcgaggc catcacgccg cagaccttga 120
   tcaacatccg gccggtggtc gccgcgatca aggagttctt cggcaccagc cagctctcgc 180
   agttcatgga ccagaacaac ccgctgtcgg gtctgaccca caagcgtcgt ctgtcggcgt 240
   tgggcccggg tggtctgtcg cgtgagcgtg ccgggctgga agtccgtgac gtgcacccgt 300
   cgcact 306
<210> 63
   <211> 306
   <212> DNA
   <213> Mycobacterium intracellulare
<400> 63
   tgcgcaccgt gggtgagctg atccagaacc agatccgggt cggcatgtcg cggatggagc 60
   gcgtcgtccg cgagcggatg accacgcagg acgtcgaggc catcacgccg cagaccctga 120
   tcaacatccg gccggtcgtc gccgcgatca aggagttctt cggcaccagc cagctgagcc 180
   agttcatgga ccagaacaac ccgctgtccg gtctgaccca caagcgccgc ctctcggcgc 240
   tgggccccgg cggtctgtcc cgtgagcgcg ccggcctgga ggtccgtgac gtccacccct 300
   cgcact 306
<210> 64
   <211> 306
   <212> DNA
   <213> Mycobacterium kansasii
<400> 64
   tgcgcaccgt gggtgagctg atccagaacc agatccgggt cggcatgtcg cggatggagc 60
   gcgtcgtccg cgagcggatg accacgcagg acgtcgaggc catcacgccg cagaccctga 120
   tcaacatccg gccggtcgtc gccgcgatca aggagttctt cggcaccagc cagctgagcc 180
   agttcatgga ccagaacaac ccgctgtccg gtctgaccca caagcgccgc ctctcggcgc 240
   tgggccccgg cggtctgtcc cgtgagcgcg ccggcctgga ggtccgtgac gtccacccct 300
   cgcact 306
<210> 65
   <211> 306
   <212> DNA
   <213> Mycobacterium leprae
<400> 65
   tgcgcacggt cggcgaattg atccagaacc agatccgggt cggtatgtcg cggatggagc 60
   gggtggtccg ggagcggatg accacccagg acgtcgaggc gatcacgccg cagacgctga 120
   tcaatatccg tccggtggtc gccgctatca aggaattctt cggcaccagc cagctgtcgc 180
   agttcatgga tcagaacaac cctctgtcgg gcctgaccca caagcgccgg ctgtcggcgc 240
   tgggcccggg tggtttgtcg cgtgagcgtg ccgggctaga ggtccgtgac gtgcaccctt 300
   cgcact 306
<210> 66
   <211> 306
   <212> DNA
   <213> Mycobacterium malmoense
<400> 66
   tgcgcacggt cggggagctg atccagaacc agatccgcgt cggcatgtcg cggatggagc 60
   gcgtcgtccg ggagcggatg accacccagg acgtcgaggc gatcacgccg cagacgctga 120
   tcaacatccg gccggtggtc gccgcgatca aggagttctt cggcaccagc cagctgtcgc 180
   agttcatgga ccagaacaac ccgctgtcgg ggctgaccca caagcgccgg ctgtcggcgc 240
   tgggcccggg tggtctgtcg cgtgagcgtg ccggcttgga ggtccgtgac gtgcacccgt 300
   cgcact 306
<210> 67
   <211> 306
   <212> DNA
   <213> Mycobacterium marinum
<400> 67
   tgcgcacggt gggtgagctg atccagaacc agatccgggt cggcatgtcg cggatggagc 60
   gggtggtccg ggagcggatg accacccagg acgtcgaggc gatcacgccg cagacgctga 120
   tcaacatccg tccggtcgtt gcggcgatca aggagttctt cggaaccagc cagctgtcgc 180
   agttcatgga ccagaacaac ccgctctccg gtctcaccca caagcgccgc ctctcggcgc 240
   tggggccggg cggtctgtcc cgtgagcgcg ccggtctgga agttcgtgac gtgcacccgt 300
   cgcact 306
<210> 68
   <211> 306
   <212> DNA
   <213> Mycobacterium avium subsp. paratuberculosis
<400> 68
   tgcgcaccgt cggtgagctg atccagaacc agatccgggt cggcatgtcc cggatggagc 60
   gcgtcgtccg cgagcggatg accacccagg acgtcgaggc catcacgccg cagaccctga 120
   tcaacatccg tcccgtcgtg gcggcgatca aggagttctt cggcaccagc cagttgtccc 180
   agttcatgga ccagaacaac ccgctgtcgg ggctcaccca caagcgccgc ctgtcggcgc 240
   tgggcccggg tggtctgtcc cgggagcgtg ccgggctgga ggtccgcgac gtgcacccgt 300
   cccact 306
<210> 69
   <211> 306
   <212> DNA
   <213> Mycobacterium scrofulaceum
<400> 69
   tgcgcaccgt cggtgagctg atccagaacc agatccgggt cggcctgtcg cgtatggagc      60
   gtgtcgtgcg tgagcgcatg accacccagg acgtcgaggc gatcaccccg cagaccctga     120
   tcaacatccg ccccgtcgtg gcggcgatca aggagttctt cggcaccagc cagctgtcgc     180
   agttcatgga ccagaacaac ccgctgtcgg gtctgaccca caagcgtcgt ctgtcggcgc     240
   tgggccccgg cggtctgtcc cgtgagcgcg ccggccttga ggtccgcgac gtgcactcca     300
   gccact                                                                306
<210> 70
   <211> 306
   <212> DNA
   <213> Mycobacterium shimoidei
<400> 70
   tgcgcacggt gggtgagctg atccagaacc agatccgggt cggcatgtcg cggatggagc      60
   gggtggtccg ggagcggatg accacccagg acgtcgaggc gatcacgccg cagacgctga     120
   tcaacatccg tccggtcgtt gccgcgatca aggagttctt cggaaccagc cagctgtcgc     180
   agttcatgga ccagaacaac ccgctgtccg gtctcaccca caagcgccgc ctctcggcgc     240
   tggggccggg cggtctgtcc cgtgagcgtg ccgggctgga agttcgtgac gtgcacccgt     300
   cgcact                                                                306
<210> 71
   <211> 306
   <212> DNA
   <213> Mycobacterium simiae
<400> 71
   tgcgcaccgt gggtgagctg atccagaacc agatccgggt cggcctgtcc cgcatggagc 60
   gcgtcgtgcg tgagcggatg accacccagg acgtcgaggc gatcacgccg cagaccctga 120
   tcaacatccg tcccgtcgtg gcggcgatca aggagttctt cggtaccagc cagctgtcgc 180
   agttcatgga ccagaacaac ccgctttcgg gtctgaccca caagcgtcgc ctgtcggcgc 240
   tgggccccgg cggtctgtcc cgtgagcgtg ccggccttga ggtccgcgac gtgcacgcca 300
   gccact 306
<210> 72
   <211> 306
   <212> DNA
   <213> Mycobacterium szulgai
<400> 72
   tgcgcaccgt gggcgagttg atccagaacc agatccgggt cggcatgtcc cggatggagc 60
   gcgtcgtgcg cgagcggatg accacccagg acgtcgaggc gatcacgccg cagaccctga 120
   tcaacatccg gcccgtcgtc gccgcgatca aggagttctt cggcaccagc cagctctcgc 180
   agttcatgga ccagaacaac ccgctctccg gtctgaccca caagcggcgt ctgtccgctc 240
   tggggccggg cggtctgtcc cgtgagcggg ccgggctgga ggtccgtgac gtgcacccgt 300
   cgcact 306
<210> 73
   <211> 306
   <212> DNA
   <213> Mycobacterium ulcerans
<400> 73
   tgcgcacggt gggtgagctg atccagaacc agatccgggt cggcatgtcg cggatggagc 60
   gggtggtccg ggagcggatg accacccagg atgtcgaggc gatcacgccg cagacgctga 120
   tcaacatccg tccggtcgtt gccgcgatca aggagttctt cggaaccagc cagctgtcgc 180
   agttcatgga ccagaacaac ccgctctccg gtctcaccca caagcgccgc ctctcggcgc 240
   tggggccggg cggtctgtcc cgtgagcgcg ccggtctgga agttcgtgac gtgcacccgt 300
   cgcact 306
<210> 74
   <211> 306
   <212> DNA
   <213> Mycobacterium xenopi
<400> 74
   tgcgcacggt cggcgagctg atccaaaacc agatccgggt cggcatgtcg aggatggagc 60
   gggtggtccg cgagcggatg accactcagg acgtcgaggc gatcaccccg cagaccttga 120
   tcaacatccg ccccgtggtg gccgcgatca aggagttctt cggcaccagc cagctctcgc 180
   agttcatgga tcagaacaac ccgctgtcgg ggctcaccca caagcggcgg ctctcggcgc 240
   ttggtccggg cggtctgtcg cgcgagcggg ccgggctgga ggtccgtgac gtgcactcga 300
   gccact 306
<210> 75
   <211> 306
   <212> DNA
   <213> Mycobacterium terrae
<400> 75
   tgcgcacggt gggtgagctg atccagaacc agatccgggt cgggttgtcc cggatggagc 60
   gtgtggtccg cgagcggatg accacccagg acgtcgaggc catcacgccg cagaccctga 120
   tcaacatccg cccggtggtc gccgcgatca aggagttctt cggcaccagc cagctctcgc 180
   agttcatgga ccagaacaac ccgctgtcgg gtctgaccca caagcgccgg ctgtcggcgc 240
   tgggcccggg tggtctgtcc cgtgagcgtg ccgggcttga ggtccgtgac gtgcacccgt 300
   cccact 306
<210> 76
   <211> 306
   <212> DNA
   <213> Mycobacterium triviale
<400> 76
   tgcgcaccgt cggggagttg atccagaacc agatccgggt cgggctgtcc cggatggagc 60
   gggtggtgcg cgagcggatg accacccagg atgtcgaggc gatcacgccg cagaccctga 120
   tcaacatccg cccggtggtc gccgcgatca aggagttctt cggcaccagc cagctgtcgc 180
   agttcatgga ccagaacaac ccgctgtccg ggctgaccca caagcgccgg ctgtcggcgc 240
   tggggcccgg cgggctctcc cgggagcggg ccgggctgga ggtccgcgac gtgcacccca 300
   gccact 306
<210> 77
   <211> 306
   <212> DNA
   <213> Mycobacterium nonchromogenicum
<400> 77
   tgcgcaccgt gggtgagctg atccagaacc agatccgggt cgggctgtcc cggatggagc 60
   gcgtggtccg cgagcggatg accacccagg acgtcgaggc catcacgccg cagaccctga 120
   tcaacatccg cccggtggtc gccgccatca aggaattctt cggcaccagc cagctgtcgc 180
   agttcatgga ccagaacaac ccgctgtcag gtctgaccca caagcggcgt ctgtcggcgc 240
   tgggccccgg tggtctgtcg cgtgagcgcg ccggcctgga agttcgtgac gtgcacccgt 300
   cccact 306
<210> 78
   <211> 306
   <212> DNA
   <213> Mycobacterium abscessus
<400> 78
   tgcgtaccgt cggcgagctg attcagaacc agatccgggt cggcctgtcc cgtatggagc 60
   gcgtcgtgcg tgagcgcatg accacgcagg acgtcgaggc gatcaccccg cagaccctga 120
   tcaacatccg tcccgtcgtg gcggcgatca aggagttctt cggaaccagc cagctgtcgc 180
   agttcatgga ccagaacaac ccgctgtcgg gcctgaccca caagcgtcgt ctgtcggcgc 240
   tgggccccgg tggtctgacc cgtgaccgcg ccggcctcga ggtccgcgac gtgcacccct 300
   cgcact 306
<210> 79
   <211> 306
   <212> DNA
   <213> Mycobacterium aurum
<400> 79
   tgcgcaccgt gggcgagttg atccagaacc agatccgggt cggcatgtcc cggatggagc 60
   gcgtcgtccg cgagcggatg accactcagg acgtcgaggc gatcacgccg cagaccctga 120
   tcaacatccg gccggtcgtt gccgcgatca aggagttctt cggcaccagc cagctctcgc 180
   agttcatgga ccagaacaac ccgctttcgg gtctgaccca caagcgccgc ctgtcggcgc 240
   tgggccccgg cggtctgtcc cgtgagcgtg ccggcctgga agtgcgtgac gtgcacccct 300
   cgcact 306
<210> 80
   <211> 306
   <212> DNA
   <213> Mycobacterium chelonae
<400> 80
   tgcgtaccgt cggcgagctg atccagaacc agatccgggt cggcctgtcg cgtatggagc      60
   gcgtcgtgcg tgagcgcatg accactcagg acgtcgaggc gatcaccccg cagaccctga     120
   tcaacatccg tcccgtcgtg gcggcgatca aggagttctt cggaaccagc cagctgtcgc     180
   agttcatgga ccagaacaac ccgctttcgg gtctgaccca caagcgtcgt ctgtcggctc     240
   tgggccccgg tggtctgacc cgtgaccgcg ctggccttga ggtccgcgac gtgcacccct     300
   cgcact                                                                306
<210> 81
   <211> 306
   <212> DNA
   <213> Mycobacterium chitae
<400> 81
   tgcgcaccgt gggtgagctg atccagaacc agatccgggt cggcctgtcc cgcatggagc      60
   gcgtcgtgcg cgagcggatg accacccagg acgtcgaggc catcacgccg cagaccctga     120
   tcaacatccg tcccgtcgtg gcggcgatca aggagttctt cggcaccagc cagctgtcgc     180
   agttcatgga ccagaacaac ccgctgtccg ggctgaccca caagcgtcgt ctctcggcgc     240
   tcgggcccgg cggtctgtcc cgtgagcgcg ccggtctcga ggttcgtgac gtgcacccgt     300
   cgcact                                                                306
<210> 82
   <211> 306
   <212> DNA
   <213> Mycobacterium fallax
<400> 82
   tgcgcaccgt gggcgagctg atccagaacc agatccgggt cggcctgtcc cggatggagc 60
   gcgtcgtccg cgagcggatg accacccagg acgtcgaggc gatcaccccg cagaccctga 120
   tcaacatccg tcccgtggtg gcggcgatca aggagttctt cgggaccagc cagctgtcgc 180
   agttcatgga ccagaacaac ccgctgtcgg gcctgaccca caagcgccgg ctgtccgcgc 240
   ttggccccgg cggtctgtcc cgtgagcgcg ccggcctgga ggtccgcgac gtgcacgcca 300
   gccact 306
<210> 83
   <211> 306
   <212> DNA
   <213> Mycobacterium flavescens
<400> 83
   tgcgcaccgt cggcgagctg atccagaacc agatccgggt cggcctgtcg cggatggagc 60
   gcgtcgtccg tgagcggatg accacccagg acgtcgaggc gatcacgccg cagaccctga 120
   tcaacatccg tcccgtcgtg gcggcgatca aggagttctt cggtacgtcg cagctgtcgc 180
   agttcatgga ccagaacaac ccgctgtcgg gtctgaccca caagcgccgc ctgtcggcgc 240
   tgggccccgg tggtctgtcc cgtgagcgcg ccggcctcga agtccgtgac gtgcacccgt 300
   cgcact 306
<210> 84
   <211> 306
   <212> DNA
   <213> Mycobacterium fortuitum strain ATCC6841
<400> 84
   tgcgcaccgt gggcgagctg atccagaacc agatccgcgt cggcctgtcc cgcatggagc    60
   gcgtcgtgcg tgagcgcatg accacccagg acgtcgaggc gatcaccccg cagaccctga   120
   tcaacatccg tcccgtcgtg gcggcgatca aggagttctt cggaacgtcg cagctgtcgc   180
   agttcatgga tcagaacaac ccgctgtcgg gtctgaccca caagcgtcgt ctgtcggcgc   240
   tgggccccgg cggtctgtcc cgtgagcgcg ccggccttga ggtccgcgac gtccactcgt   300
   cgcact                                                              306
<210> 85
   <211> 306
   <212> DNA
   <213> Mycobaterium fortuitum strain ATCC49403
<400> 85
   tgcgcaccgt gggcgagctg atccagaacc agatccgggt cggcctgtcc cgcatggagc    60
   gcgtcgtgcg tgagcgcatg accacccagg acgtcgaggc gatcaccccg cagaccctga   120
   tcaacatccg tcccgtcgtg gcggcgatca aggagttctt cggaacgtcg cagctgtcgc   180
   agttcatgga tcagaacaac ccgctgtcgg gtctgaccca caagcgtcgt ctgtcggcgc   240
   tgggccccgg cggtctgtcc cgtgagcgcg ccggccttga ggtccgcgac gtccactcgt   300
   cgcact                                                              306
<210> 86
   <211> 306
   <212> DNA
   <213> Mycobacterium neoaurum
<400> 86
   tgcgcaccgt gggtgagctg atccagaatc agatccgggt cggcctgtcg cgcatggagc 60
   gggtcgtgcg cgagcgcatg accacccagg acgtcgaggc gatcaccccg cagaccctga 120
   tcaacatccg tcccgtcgtg gcggcgatca aggagttctt cgggaccagc cagctgtcgc 180
   agttcatgga ccagaacaac ccgctgtcgg gtctgaccca caagcgtcgt ctgtcggcgc 240
   tgggccccgg tggtctgtcc cgtgagcgtg ccggacttga ggtccgcgac gtgcactcca 300
   gccact 306
<210> 87
   <211> 306
   <212> DNA
   <213> Mycobacterium peregrinum
<400> 87
   tgcgcaccgt cggtgagctg atccagaacc agatccgggt cggcctgtcg cgtatggagc 60
   gtgtcgtgcg tgagcgcatg accacccagg acgtcgaggc gatcaccccg cagaccctga 120
   tcaacatccg ccccgtcgtg gcggcgatca aggagttctt cggcaccagc cagctgtcgc 180
   agttcatgga ccagaacaac ccgctgtcgg gtctgaccca caagcgtcgt ctgtcggcgc 240
   tgggccccgg cggtctgtcc cgtgagcgcg ccggccttga ggtccgcgac gtgcactcca 300
   gccact 306
<210> 88
   <211> 306
   <212> DNA
   <213> Mycobacterium phlei
<400> 88
   tgcgcaccgt cggcgagctg atccagaacc agatccgggt cggcctgtcg cgtatggagc      60
   gcgtcgtgcg cgagcgcatg accacccagg acgtcgaggc gatcacgccg cagaccctga     120
   tcaacatccg tcccgtcgtg gcggcgatca aggagttctt cggcaccagc cagctgtcgc     180
   agttcatgga ccagaacaac ccgctgtcgg gtctgaccca caagcgccgc ctgtcggcgc     240
   tgggcccggg cggtctgtcc cgtgagcgcg ccggcctcga ggtccgcgac gtgcaccaca     300
   gccact                                                                306
<210> 89
   <211> 306
   <212> DNA
   <213> Mycobacterium senegalense
<400> 89
   tgcgcaccgt gggtgagctg atccagaacc agatccgggt cggcctgtcc cgcatggagc      60
   gcgtcgtgcg tgagcggatg accacccagg acgtcgaggc gatcacgccg cagaccctga     120
   tcaacatccg tcccgtcgtg gcggcgatca aggagttctt cggtaccagc cagctgtcgc     180
   agttcatgga ccagaacaac ccgctttcgg gtctgaccca caagcgtcgc ctgtcggcgc     240
   tgggccccgg cggtctgtcc cgtgagcgtg ccggccttga ggtccgcgac gtgcacgcca     300
   gccact                                                                306
<210> 90
   <211> 306
   <212> DNA
   <213> Mycobacterium smegmatis
<400> 90
   tgcgcaccgt cggtgagctg atccagaacc agatccgcgt gggcctgtcc cgcatggagc 60
   gtgtcgtgcg tgagcgcatg accacccagg acgtcgaggc gatcacgccg cagaccctga 120
   tcaacatccg tcccgtcgtg gcggcgatca aggagttctt cggcaccagc cagctgtcgc 180
   agttcatgga ccagaacaac ccgctgtcgg gtctgaccca caagcgtcgt ctttcggcgc 240
   tgggccccgg cggtctgtcc cgtgagcgcg ctggcctcga ggtccgcgac gtgcacccca 300
   gccact 306
<210> 91
   <211> 306
   <212> DNA
   <213> Mycobacterium thermoresistibile
<400> 91
   tgcgcaccgt cggcgagctg atccagaacc agatccgggt cggcctgtcc cgcatggagc 60
   gcgtcgtgcg cgagcggatg accacccagg acgtcgaggc gatcacgccg cagaccctga 120
   tcaacatccg ccccgtcgtg gcggcgatca aggagttctt cggcaccagc cagctgagcc 180
   agttcatgga ccagaacaac ccgctgtcgg gtctgaccca caagcgccgg ctgtcggcgc 240
   tgggcccggg cggtctgagc cgggagcgcg ccggcctcga ggtccgcgac gtccacccgt 300
   cgcact 306
<210> 92
   <211> 306
   <212> DNA
   <213> Mycobacterium vaccae
<400> 92
   tgcgcacggt cggtgagctg atccagaacc agatccgcgt cggcctctcg cgtatggagc 60
   gtgtcgtccg cgagcggatg accacccagg acgtcgaggc gatcactccg cagaccctga        120
   tcaacatccg tcccgtcgtg gcggcgatca aggaattctt cggcaccagc cagctgtcgc        180
   agttcatgga ccagaacaac ccgctgtcgg gtctgaccca caagcgtcgc ctgtcggcgc        240
   tgggccccgg cggtctgtcc cgtgagcgcg ccggcctcga ggtccgcgac gtgcactcca        300
   gccact                                                                   306